(19) Europäisches Patentamt / European Patent Office / Office européen des brevets

(11) **EP 3 407 866 B1**

(12) **FASCICULE DE BREVET EUROPEEN**

(45) Date de publication et mention de la délivrance du brevet:
**14.04.2021 Bulletin 2021/15**

(21) Numéro de dépôt: **17704683.6**

(22) Date de dépôt: **27.01.2017**

(51) Int Cl.:
*A61K 8/73* (2006.01)    *A61Q 17/00* (2006.01)
*A61Q 19/08* (2006.01)    *A61K 36/04* (2006.01)
*A61K 36/48* (2006.01)    *A61P 39/00* (2006.01)
*A61K 8/9717* (2017.01)    *A61K 8/9789* (2017.01)
*A61K 8/9794* (2017.01)

(86) Numéro de dépôt international:
**PCT/EP2017/051827**

(87) Numéro de publication internationale:
**WO 2017/129780 (03.08.2017 Gazette 2017/31)**

(54) **AGENT COSMETIQUE CONSTITUE PAR DES GALACTOMANNANES OBTENUS A PARTIR DE CAESALPINIA SPINOSA ET DES GALACTANES SULFATES RETICULES OBTENUS A PARTIR DE KAPPAPHYCUS ALVAREZII**

KOSMETISCHES MITTEL BESTEHEND AUS GALACTOMANNANEN AUS CAESALPINIA SPINOSA UND VERNETZTEN SULFATIERTEN GALACTANEN AUS KAPPAPHYCUS ALVAREZII

COSMETIC AGENT CONSISTING OF GALACTOMANNANS OBTAINED FROM CAESALPINIA SPINOSA AND CROSS-LINKED SULPHATED GALACTANS OBTAINED FROM KAPPAPHYCUS ALVAREZII

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **29.01.2016 FR 1650741**

(43) Date de publication de la demande:
**05.12.2018 Bulletin 2018/49**

(73) Titulaire: **Societe Industrielle Limousine d'Application Biologique**
**19130 Objat (FR)**

(72) Inventeur: **PAUFIQUE, Jean**
**19130 Objat (FR)**

(74) Mandataire: **Aquinov**
**Allée de la Forestière**
**33750 Beychac et Caillau (FR)**

(56) Documents cités:
EP-A2- 0 730 867    WO-A1-84/04039
WO-A1-98/19663    FR-A1- 2 881 349
FR-A1- 2 882 366    FR-A1- 2 986 430
FR-A1- 3 018 448

• M D Guiry ET AL: "Kappaphycus alvarezii (Doty) Doty ex P.C.Silva :: Algaebase", AlgaeBase, 26 février 2013 (2013-02-26), pages 1-4, XP055282920, ireland Extrait de l'Internet: URL:http://www.algaebase.org/search/species/detail/?species_id=W5ac9a714e03445eb&sk=0&from=results [extrait le 2016-06-22]
• DATABASE GNPD [Online] MINTEL; 1 juin 2014 (2014-06-01), Laboratorium Kosmetyczne Floslek: "Dermal Filler Day Cream SPF 15", XP002759145, Database accession no. 2482521
• DATABASE GNPD [Online] MINTEL; 1 novembre 2009 (2009-11-01), Nazca Cosmeticos: "Leave-in Cream", XP002759146, Database accession no. 1208217
• Anonymous: "Hydromanil", , 1 janvier 2005 (2005-01-01), pages 1-17, XP055115308, Extrait de l'Internet: URL:http://www.innovadex.com/documents/524153.pdf?bs=2443&b=71056&st=1&sl=28547834&c rit=a2V5d29yZDpbaHlkcm9tYW5pbF0=&k=hydroma nil [extrait le 2014-04-25]

**EP 3 407 866 B1**

**Description**

La présente invention concerne un agent cosmétique particulier constitué par des galactomannanes obtenus à partir de *Caesalpina spinosa* et des galactanes sulfatés réticulés obtenus à partir de *Kappaphycus alvarezii,* présentant un effet tenseur et/ou filmogène et son utilisation pour des applications cosmétiques ou dermocosmétiques.

Les femmes sont en quête perpétuelle de solutions visant à garder leur peau jeune et en bonne santé. Elles recherchent le produit capable d'effacer les signes du temps et de leur permettre de se protéger des effets néfastes d'un environnement toujours plus agressif.

En effet, en raison de l'industrialisation et du grossissement des villes, les individus sont régulièrement exposés à des allergènes, des molécules irritantes ou des particules fines. Ces dernières, notamment émises par les véhicules, ont des effets dévastateurs sur la santé. Au-delà de leur incidence au niveau respiratoire, elles entrainent des dégâts majeurs sur la peau en accélérant son vieillissement : apparition de rides et de taches pigmentaires, peau relâchée, perte d'élasticité et manque d'éclat.

Face à cette problématique, le marché des cosmétiques propose une large gamme de soins anti-âge et protecteur. On trouve des produits visant à remodeler en profondeur et sur le long terme la peau afin de lui redonner sa jeunesse. De plus, un éventail d'antioxydants, de détoxifiants ou de stimulateurs des défenses naturelles est à disposition pour aider la peau à se protéger.

Toutefois, en complément de ces traitements « longue durée », les consommateurs souhaitent aussi des résultats immédiats. Les soins intègrent donc dorénavant et très largement des actifs liftants capables d'améliorer instantanément les signes du vieillissement. Depuis 2010, on voit progresser dans les soins de peau, une nouvelle catégorie d'actifs : les actifs « seconde peau ». Ces derniers dotés d'une action à court et long terme sont intégrés pour leur action coup d'éclat et protecteur face aux agressions extérieures.

L'objectif de la présente invention est de proposer un nouvel agent cosmétique avec un effet tenseur perceptible et un effet film « seconde peau » protecteur et liftant capable de booster la jeunesse cutanée.

A cet effet, l'invention vise un agent cosmétique ou dermocosmétique constitué par l'association de biopolymères spécifiques, des galactomannanes obtenus à partir de *Caesalpinia spinosa* de masses molaires comprises entre 1 et 150kDa, et des galactanes sulfatés réticulés obtenus à partir de *Kappaphycus alvarezii* de masses molaires comprises entre 1 et 150kDa.

Les galactomannanes sont connus comme étant des émulsifiants, des épaississants largement utilisés en cosmétique et en alimentaire. Ce sont des polysaccharides d'une taille très élevée (environ 3000kDa). La viscosité de ces polysaccharides est proportionnelle à la taille des polysaccharides, et elle apporte l'effet d'épaississement. La gomme de *Caesalpinia spinosa* est connue dans les compositions cosmétiques. Elle est généralement utilisée pour son pouvoir épaississant. On trouve aussi des compositions utilisant la combinaison de la gomme de *Caesalpinia spinosa* et d'oligosaccharides de *Caesalpinia spinosa,* commercialisé sous la marque Hydromanil (innovadex.com). L'utilisation de galactomannanes de caesalpinia spinosa est également décrite dans la publication de Nazca cosmeticos : « Leave-in cream » du 1er novembre 2009. Des Galactomannanes sont également décrits en tant qu'agent actif en cosmétique dans FR2881349. Tous ces produits connus sont différents de l'agent selon l'invention et ne présentent pas d'efficacité tenseur et filmogène.

Dans l'art antérieur, les kappa carraghénanes, contenant des galactanes sulfatés, sont connus comme étant des émulsifiants, des épaississants largement utilisés en cosmétique et en alimentaire. Ce sont des polysaccharides d'une taille très élevée (jusqu'à 20000kDa). La viscosité de ces polysaccharides est proportionnelle à la taille des polysaccharides, et elle apporte l'effet d'épaississement. On peut aussi trouver des ingrédients cosmétiques issus de l'hydrolyse de *kappaphycus alvarezii,* comme le produit décrit dans la demande FR2986430. De même, la publication Laboratorium Kosmetyczne Floslek « Dermal Filler Day Cream SPF 15 » du 1er juin 2014 décrit des galactanes de chondrus crispus. Les documents FR2986430, EP0730867, WO98/19663, FR3018448 et WO84/04039 décrivent aussi l'utilisation de galactanes sulfatés. Par ailleurs, le document FR2882366 décrit des polymères réticulés de carbohydrates et leur utilisation en cosmétique. Enfin, MD Guiry et al. « Kappaphycus alvarezii (Doty) Doty ex P.C. Silva : : Algaebase » AlgaeBase, 26 février 2013, pages 1-4, donne des informations générales sur Kappaphycus alvarezii. L'invention concerne l'association de galactomannanes obtenus à partir de *Caesalpinia spinosa* préférentiellement de masse molaire moyenne comprise entre 5 et 30kDa et de galactanes sulfatés réticulés obtenus à partir de *Kappaphycus alvarezii* préférentiellement de masse molaire moyenne comprise entre 5 et 25kDa. Avantageusement, l'agent selon l'invention présente des propriétés biomécaniques et filmogènes puissantes qui confèrent une efficacité de seconde peau protectrice. Avantageusement, ils sont réalisés sans utilisation d'agents chimiques et répondent aux exigences d'écoconception. Avec cette association particulière de biopolymères, la peau est protégée, elle retrouve éclat et attractivité. Les signes visibles du vieillissement de la peau sont instantanément gommés. Avec cette association particulière de biopolymères, la peau est protégée des polluants, irritants, allergènes et métaux lourds. La barrière cutanée naturelle de la peau est renforcée puisqu'elle diminue la pénétration de ces molécules toxiques.

L'invention a donc également pour objet l'utilisation cosmétique de cet agent cosmétique. L'invention vise aussi les compositions cosmétiques incluant l'agent cosmétique selon l'invention ainsi qu'un procédé de traitement cosmétique de la peau utilisant ces compositions.

D'autres caractéristiques et avantages ressortiront de la description en détail de l'invention qui va suivre en regard des figures annexées sur lesquelles :

- La Figure 1A représente le chromatogramme du produit décrit dans la demande FR2986430,
- La Figure 1B représente le chromatogramme d'un composant B1,
- La Figure 1C représente le chromatogramme d'un agent selon l'invention;
- les Figures 2A à 2E représentent l'échelle de « - » (Figure 2E) à « ++++ » (Figure 2A) permettant d'évaluer la force de rétractation d'un polymère;
- la Figure 3 représente l'image AFM (Microscope de Force Atomique) d'un agent selon l'invention ;
- les Figures 4A à 4C représentent les courbes DSC (calorimétrie différentielle à balayage) pour des galactanes sulfatés sélectionnés selon l'invention (Figure 4A), pour des galactomannanes sélectionnés selon l'invention (Figure 4B) et pour une agent selon l'invention (Figure 4C) ;
- les Figures 5A à 5D représentent les courbes DMA (Analyse mécanique dynamique) pour des galactomannanes sélectionnés selon l'invention (Figures 5A-5B) et pour un agent selon l'invention (Figures 5C-5D),
- la Figures 6A représente le biofilm de *Staphylococcus aureus* sur l'épiderme reconstruit témoin,
- La Figure 6B représente le biofilm de *Staphylococcus aureus* sur l'épiderme reconstruit traité par l'agent selon l'invention.

## DEFINITIONS

Par « agent cosmétique ou dermocosmétique » au sens de l'invention, aussi noté « agent », on entend un principe actif avec un effet cosmétique adapté à une utilisation topique dans une composition cosmétique ou dermocosmétique.

L'agent selon l'invention est constitué de biopolymères, et peut-être également désigné dans le présente demande par le terme « biopolymère ».

Par « biopolymères», au sens de l'invention, on entend des polymères issus de matières premières végétales, par opposition aux polymères synthétiques, qui sont obtenus par synthèse chimique.

« Par booster de beauté » au sens de l'invention, on entend un agent qui améliore l'attractivité du visage perçue par un tiers. L'attractivité est appréciée sur l'amélioration globale du visage par comparaison de photographies avant et après traitement.

Par « filmogène », au sens de l'invention, on entend un biopolymère présentant un effet filmogène, c'est-à-dire un biopolymère soluble à la concentration de 7% en poids dans l'eau ou à la concentration maximale à laquelle il forme un milieu d'apparence homogène et produisant une fois séché pendant 24h à 40°C, un film qui nécessite une masse d'au moins 100g pour le rompre dans le test décrit dans la présente demande.

Par « effet filmogène » on entend un effet pouvant créer à la surface de la peau un film non perceptible à l'œil nu, et ainsi protéger la peau des agressions externes telle que la pollution et les allergènes.

Par « effet tenseur », on entend un effet de tension sur la peau et par cet effet de tension, lisser la peau, diminuer les pores de la peau et faire diminuer de façon immédiate les rides et ridules.

Par « tenseur », au sens de l'invention, on entend un biopolymère présentant un effet tenseur, c'est-à-dire tout biopolymère soluble à la concentration de 7% en poids dans l'eau ou à la concentration maximale à laquelle il forme un milieu d'apparence homogène et produisant à cette concentration, une rétractation notée au moins « +++ » dans le test décrit dans la présente demande.

Par « masse molaire moyenne » d'un mélange de molécules au sens de l'invention on entend la moyenne des masses molaires pondérales de chaque molécule du mélange. On entend par « milieu d'apparence homogène » un milieu ne présentant pas d'agrégats visibles à l'œil nu.

Par « réticulé » au sens de l'invention, on entend un biopolymère dans lequel a été formé un réseau tridimensionnel au moyen de la formation de liaisons chimiques ou physiques entre les molécules du biopolymère.

## DESCRIPTION DETAILLEE DE L'INVENTION

L'invention vise donc un agent cosmétique ou dermocosmétique constitué par :

- des galactomannanes obtenus à partir de *Caesalpinia spinosa* de masses molaires comprises entre 1 et 150kDa,
- et des galactanes sulfatés réticulés obtenus à partir de *Kappaphycus alvarezii* de masses molaires comprises entre 1 et 150kDa, préférentiellement entre 7 et 40kDa.

Il s'agit donc d'un agent constitué de biopolymères particuliers, sélectionnés, avec des caractéristiques spécifiques.

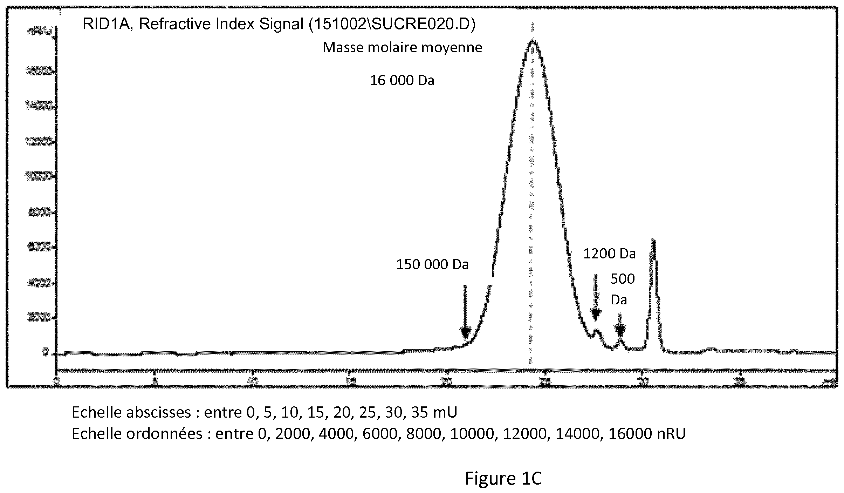

La détermination des masses molaires de ces biopolymères de nature saccharidique, est préférentiellement réalisée par chromatographie d'exclusion stérique. Cette méthode de chromatographie liquide permet de séparer les macromolécules en fonction de leur volume hydrodynamique (chromatographie d'exclusion stérique). Les solutés sont élués dans l'ordre des masses molaires décroissantes après passage sur 3 colonnes de perméation sur gel montées en série (colonnes PL aquagel-OH C60, C40 et C30). Les composés sont détectés par un détecteur à indice de réfraction. Les masses molaires des carbohydrates sont évaluées par comparaison des temps de rétention des pics détectés dans les échantillons des agents selon l'invention avec les temps de rétention de standards injectés au préalable. La masse molaire moyenne d'un mélange de molécules correspond à la moyenne des masses molaires pondérée par l'intensité de chacune. Par exemple, l'association de biopolymères dont le chromatogramme est représenté à la Figure 1C contient des polysaccharides de masses molaires comprises entre 1,2 à 150kDa, et présente une masse molaire moyenne de 16kDa.

D'un point de vue physique, l'agent de l'invention présente une faible viscosité. La viscosité est mesurée à température ambiante à l'aide d'un viscosimètre Brookfield Modèle DV-I+. Le viscosimètre Brookfield détermine la viscosité d'un fluide à partir de la déformation exercée sur un ressort créé par la rotation d'un disque dans ce fluide. On considère qu'un produit est visqueux, si sa viscosité excède 1000 centipoises.

Par ailleurs, cet agent présente un effet tenseur. Cet effet tenseur peut être défini par la caractérisation de la force de rétractation sur le modèle de peau synthétique ou par une étude sensorielle sur un panel d'experts.

La force de rétractation du biopolymère peut être caractérisée par un test in vitro. Ce modèle est connu de l'homme de métier et a été décrit dans la demande de brevet EP1944065. Le test est réalisé sur une solution homogène de biopolymères dilués dans l'eau à une concentration de 7% en poids. Le mélange homogène est déposé sur un film de peau synthétique d'une épaisseur d'environ 100 $\mu$m et de largeur initiale de 10mm. Après séchage à 22 $\pm$ 3°C et 40 $\pm$ 10% d'humidité relative, la peau synthétique présente une largeur rétractée due à la tension exercée par le polymère déposé.

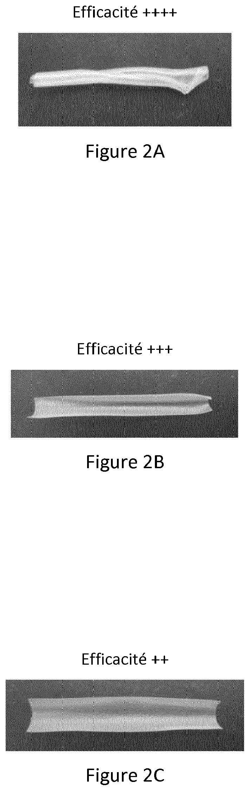

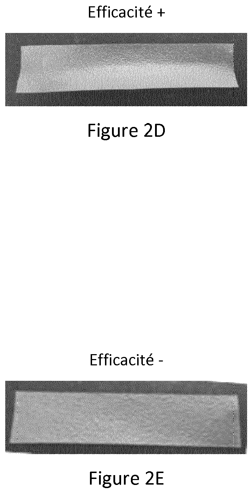

Cette rétractation est évaluée visuellement et quantifiée sur une échelle de « - » à « ++++ » (« - » pas d'effet tenseur, « ++++ »: effet tenseur maximal) représentée sur les Figures 2A à 2E.

Un produit est considéré comme tenseur avec ce test, si la rétractation est notée au moins « ++ ».

L'effet tenseur peut être évalué également par une étude sensorielle sur un panel d'experts. L'étude peut être réalisée en formulant les agents à tester en gel.

La perception de ces effets est appréciée par des experts sensoriels grâce à une évaluation sur une échelle de score allant de 1 à 10.

Chaque expert note sur une échelle allant de 0 à 10 (0 : pas d'effet tenseur perçu, 10 : effet tenseur important) l'intensité de la sensation perçue après l'application d'un produit (agent selon l'invention ou placebo) au niveau de la patte d'oie. La notation est réalisée 3 minutes, 5 minutes et 10 minutes après l'application du produit.

La moyenne des scores obtenus à chaque temps est calculée pour chaque expert.

Un produit est considéré comme tenseur, si la moyenne des scores obtenus est supérieure à 3.

Enfin, l'agent selon l'invention présente un effet filmogène. Cet effet filmogène peut être caractérisé au moyen d'un texturomètre. Le principe est de quantifier le poids à appliquer pour entraîner la rupture du film des échantillons à tester.

Les échantillons sont préparés comme suit :

- Séchage des solutions contenant les biopolymères à 7% (w/w) pendant 24h à 40°C
- Obtention de films d'une épaisseur entre 30 et 40$\mu$m.

L'échantillon à tester est déposé à la surface d'une mousse souple et déformable, qui permet d'imposer une déformation importante au film de biopolymères. Le substrat est constitué d'une mousse uréthane élastomère de 5 mm d'épaisseur, il mime la surface et l'élasticité de la peau.

L'étude peut être réalisée avec un Texturomètre TA-XTplus de la société Stable Micro System.

Un poinçon cylindrique exerce une contrainte mécanique sur l'échantillon à une vitesse de déplacement constante.

Une courbe du poids exercé (g) en fonction du temps (sec) est obtenue, à partir de laquelle il est possible de déterminer la masse nécessaire pour obtenir la rupture.

La masse nécessaire pour rompre les échantillons, dépend des propriétés viscoélastiques de chaque échantillon. On considère qu'un produit est filmogène si la masse exercée pour rompre le film est supérieure à 100g.

L'agent cosmétique ou dermocosmétique selon l'invention contient l'association de galactomannanes sélectionnés de *Caesalpinia spinosa* et de galactanes sulfatés réticulés sélectionnés de *Kappaphycus alvarezii*.

L'agent selon l'invention est composé des galactomannanes préférentiellement sélectionnés par leur masse molaire moyenne comprise entre 5 et 30kDa, c'est-à-dire qu'ils sont obtenus préférentiellement par transformation de galactomannanes natifs de *Caesalpinia spinosa,* préférentiellement par hydrolyse.

Ces galactomannanes sélectionnés présentent encore plus préférentiellement une masse molaire moyenne comprise entre 8 et 25kDa.

Les galactomannanes particuliers sélectionnés selon l'invention sont réalisés comme suit :

- Solubilisation de poudre de galactomannanes natifs de *Caesalpinia spinosa* dans l'eau à raison d'au moins 20g/l,
- Hydrolyse ménagée par voie chimique ou enzymatique ; la taille des biopolymères est inversement proportionnelle à la durée de l'hydrolyse ou à la concentration d'enzymes ou d'agent chimique utilisé,
- Séparation des phases soluble et insoluble, afin d'éliminer la phase insoluble,
- Sélection par filtration(s) membranaire(s) des galactomannanes de masse molaire moyenne comprise entre 5kDa et 30kDa.

On obtient un produit A liquide peu visqueux contenant des galactomannanes sélectionnés ayant une masse molaire moyenne comprise entre 5 et 30kDa, préférentiellement entre 8kDa et 25kDa.

La taille des galactomannanes sélectionnés est déterminée par chromatographie d'exclusion stérique.

L'effet tenseur est évalué sur un modèle de rétractation sur peau synthétique ou par le panel d'experts sensoriels.

La viscosité est déterminée par un viscosimètre.

La masse provoquant la rupture du film réalisé avec l'agent est évaluée par un texturomètre.

Les résultats caractéristiques de plusieurs exemples de galactomannanes obtenus à partir de *Caesalpinia spinosa,* de masse molaire moyenne comprise entre 5 et 30kDa, d'une part et de galactomannanes de masse molaire moyenne en dehors de celle objet de l'invention d'autre part, sont présentés dans le Tableau 1 ci-dessous :

***Tableau 1***

| Masses molaires mini et maxi | Masse molaire moyenne | Viscosité (CP) ou état physique | Effet tenseur sur modèle de rétractation | Effet tenseur sensoriel | Masse entrainant la rupture du film (g) |
|---|---|---|---|---|---|
| | 4,7kDa | Liquide | - | | |
| 0.5 à 40kDa | 5,4kDa | 70 | + | NT** | NT** |
| | 7,2kDa | Liquide | + | | |
| 0.7 à 62kDa | 9,5kDa | Liquide | ++ | | |
| | 10kDa | 85 | +++ | NT** | NT** |
| 0.9 à 117kDa | 13,5kDa | Liquide | +++ | | |
| | 18kDa | 90 | +++ | 4.5 | 280 |
| 1.2 à 171kDa | 19kDa | Liquide | +++ | | |
| 1.5 à 215kDa | 22kDa | Liquide | +++ | | |
| 1.6 à 160kDa | 25kDa | liquide | +++ | | |
| | 28,8kDa | Légèrement visqueux | + | NT** | NT** |
| | 38kDa | visqueux | - | | |
| Gomme Tara non hydrolysée | 1980kDa | >3125 | - | NT** | NT** |
| NM* : non mesuré ; NT** : non testé | | | | | |

Ces résultats montrent bien que les galactomannanes natifs de haute masse molaire moyenne (> 30kDa) et les galactomannanes de faible masse molaire moyenne (<5kDa) ne présentent pas l'effet tenseur recherché sur le modèle de rétractation.

L'effet tenseur visualisé sur le modèle de rétractation sur peau synthétique est corrélé par l'évaluation de l'effet tenseur perçu par un panel d'experts sensoriels. Ceci confirme que les galactomannanes sélectionnés selon l'invention, peuvent être détectés comme tenseur sur le modèle de rétractation, ou par un panel d'experts sensoriels ou par l'effet filmogène recherché mesuré au texturomètre.

Les galactomannanes avec une masse molaire moyenne comprise entre 5 et 30kDa, préférentiellement entre 8 et 25kDa présentent bien un effet tenseur et filmogène.

En plus de ces galactomannanes, l'agent cosmétique ou dermocosmétique selon l'invention contient des galactanes sulfatés réticulés sélectionnés par leur masse molaire moyenne comprise entre 5 et 25kDa. Ils sont obtenus par transformation de galactanes sulfatés natifs de *Kappaphycus alvarezii,* préférentiellement par hydrolyse.

Dans l'invention, les galactanes sulfatés particuliers, sélectionnés sont réalisés comme suit :

- Solubilisation de poudre de galactanes sulfatés natifs de *Kappaphycus alvarezii* dans l'eau à raison d'au moins 20g/l,
- Hydrolyse ménagée enzymatique ou chimique ; la taille des biopolymères est inversement proportionnelle à la durée de l'hydrolyse ou à la concentration d'enzyme ou d'agent chimique utilisé,
- Séparation des phases soluble et insoluble, afin d'éliminer la phase insoluble,
- Sélection des galactanes sulfatés de masse molaire moyenne comprise entre 5kDa et 25kDa, par filtration(s) membranaire(s).

On obtient un produit B1 liquide peu visqueux contenant des galactanes sulfatés sélectionnés ayant une masse molaire moyenne comprise entre 5kDa et 25kDa.

Ces galactanes sulfatés sélectionnés par leurs masses molaires sont réticulés par un agent réticulant, préférentiellement un agent réticulant de nature ionique. L'agent de réticulation ionique est choisi parmi des cations mono ou multivalents. D'autres agents de réticulation ionique connus de l'homme de métier peuvent être envisagés.

On obtient alors un produit B2 liquide peu visqueux contenant des galactanes sulfatés réticulés sélectionnés ayant une masse molaire moyenne comprise entre 5kDa et 25kDa. La caractérisation de la taille des galactanes sulfatés est réalisée par chromatographie d'exclusion stérique.

L'effet tenseur est évalué sur le modèle de rétractation sur peau synthétique ou par le panel d'experts sensoriels.

La viscosité est déterminée par un viscosimètre.

Les résultats caractéristiques de plusieurs exemples de galactanes sulfatés obtenus à partir de *Kappaphycus alvarezii* de masse molaire moyenne comprise entre 5 et 25kDa d'une part et de masse molaire moyenne en dehors de celle objet de l'invention sont présentés dans le Tableau 2a.

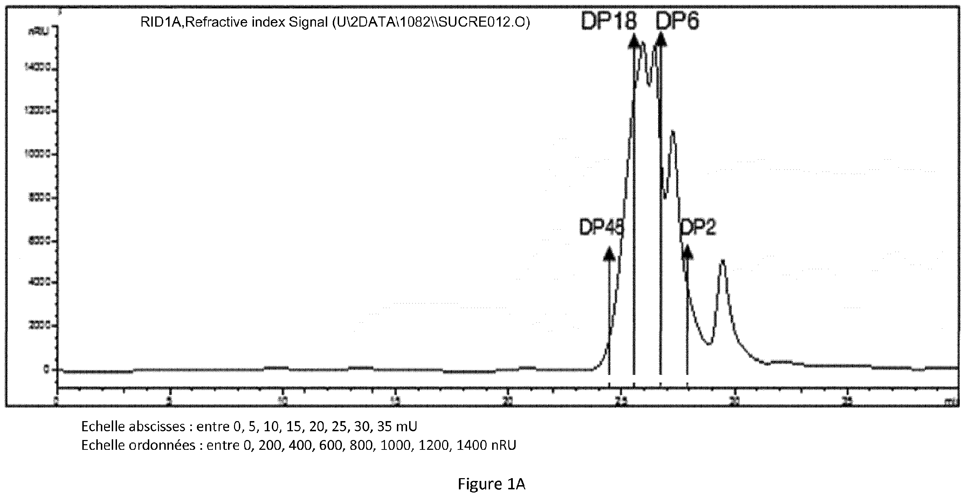

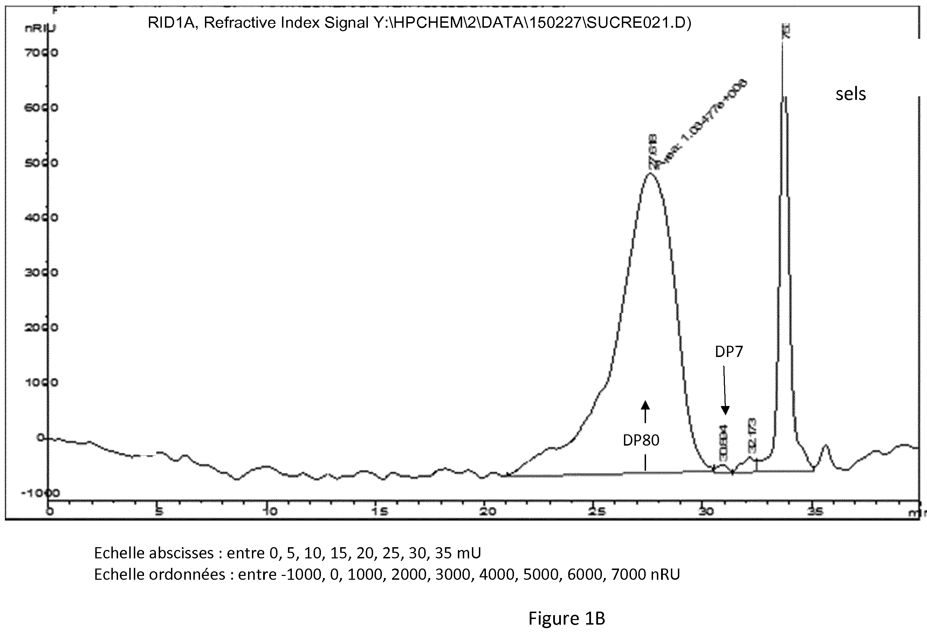

Les profils chromatographiques de deux essais sont présentés dans les Figures 1A et 1B. Les résultats caractéristiques de plusieurs exemples de galactanes sulfatés réticulés sont présentés dans le Tableau 2b.

*Tableau 2a*

| Masses molaires mini-maxi | Masse molaire moyenne | Viscosité (CP) ou état physique | Effet tenseur sur modèle de rétractation | Effet tenseur sur panel sensoriel |
|---|---|---|---|---|
| 0.18 à 8.1kDa (figure 1A) produit FR2986430 | < 3.24kDa | liquide | - | |
| | 3,6kDa | 2,2 | - | NT** |
| 0.9 à 36.3kDa | 5,4kDa | 15 | + | NT** |
| 1.3 à 66.1kDa | 10,8kDa | 300 | +++ | 3.6 |
| 1.8kDa à 150kDa (figure 1B) | 14.4 kDa | liquide | +++ | |
| 2.2 à 97.5kDa | 18kDa | liquide | ++ | NT** |
| | 25kDa | Peu visqueux | ++ | |
| | 30kDa | visqueux | + | |
| | 40kDa | visqueux | + | |
| Kappa carraghénanes non hydrolysés | 1500kDa | >3125 | - | NT** |
| NM* : non mesuré, NT** : non testé | | | | |

Le profil chromatographique de la Figure 1a permet de visualiser que les carbohydrates ont des masses molaires comprises entre 0.18kDa (DP1) à 8.1kDa (DP48), et que la masse molaire moyenne de cet essai est bien inférieure à 3.24kDa (DP18).

Le profil chromatographique de la figure 1b permet de visualiser que les carbohydrates ont des masses molaires comprises entre 1.8kDa (DP7) et 150kDa (DP833), et que la masse molaire moyenne de cet essai est bien de 14.4kDa (DP80).

Les résultats décrits dans le Tableau 2a montrent que les galactanes sulfatés de masse molaire moyenne importante (≥ 30kDa) et les galactanes sulfatés de faible masse molaire moyenne (<5kDa) ne présentent pas l'état physique ou l'effet tenseur recherchés.

Les galactanes sulfatés avec une masse molaire moyenne comprise entre 5 et 25kDa, préférentiellement entre 8 et 20kDa présentent bien l'état physique recherché et un effet tenseur visualisé soit par une force de rétraction, soit validé par les experts sensoriels.

*Tableau 2b*

| Masse molaire moyenne | Réticulation | Viscosité (CP) | Effet tenseur sur modèle de rétractation | Effet tenseur sur panel sensoriel |
|---|---|---|---|---|
| Produit FR2986430 < 3.24kDa | Non | Liquide | - | NT** |
| | Oui - agent 1 | Liquide | - | NT** |
| | Oui - agent 2 | Liquide | - | NT** |
| | Oui - agent 3 | liquide | - | NT** |
| 3,6kDa | Non | 2,2 | - | NT** |
| | Oui - agent 1 | liquide | - | NT** |
| | Oui - agent 2 | liquide | - | NT** |
| | Oui - agent 3 | liquide | - | NT** |
| 10,8kDa | Non | 300 | +++ | 3.6 |
| | Oui - agent 1 | liquide | +++ | NT** |
| | Oui - agent 2 | liquide | +++ | NT** |
| | Oui - agent 3 | liquide | ++++ | 4.5 |
| | Oui - agent 4 | liquide | ++++ | NT** |
| 18kDa | Non | liquide | ++ | NT** |
| | Oui - agent 3 | liquide | +++ | NT** |
| NT** : non testé<br>Agent 1 et 2 sont des agents de réticulation ionique type cations bivalents<br>Agent 3 est un agent de réticulation ionique type cation monovalent<br>Agent 4 est un agent de réticulation ionique type cation multivalent | | | | |

La réticulation ionique des fonctions sulfates potentialise l'effet tenseur des galactanes sulfatés sélectionnés, mais ne confère pas un effet tenseur à des galactanes sulfatés de masse molaire moyenne non sélectionnée selon l'invention.

Les galactanes sulfatés réticulés avec une masse molaire moyenne comprise entre 5 et 25kDa, préférentiellement entre 8 et 20kDa présentent bien un effet tenseur visualisé par une force de rétraction. L'effet tenseur visualisé sur le modèle de rétractation sur peau synthétique est corrélé avec l'évaluation de l'effet tenseur perçu par un panel d'experts sensoriels.

L'agent cosmétique ou dermocosmétique selon l'invention est constitué à la fois par des galactomannanes particuliers et des galactanes sulfatés réticulés particuliers, tels que décrits précédemment.

Le procédé de réalisation de l'agent selon l'invention comprend les étapes suivantes :

- obtention des galactomannanes de masse molaire moyenne comprise entre 5 et 30kDa, noté produit A selon le protocole décrit précédemment,
- obtention des galactanes sulfatés réticulés de masse molaire moyenne comprise entre 5 et 25kDa, noté produit B2 selon le protocole décrit précédemment,
- mélange du produit A et du produit B2.

Une étape de filtration peut être ajoutée après le mélange.

Selon un mode de réalisation particulièrement adapté, l'agent cosmétique ou dermocosmétique selon l'invention est constitué :

- entre 60 et 90% par les galactomannanes, et
- entre 10 et 40% par les galactanes sulfatés réticulés.

Encore plus préférentiellement, l'agent cosmétique ou dermocosmétique selon l'invention est constitué :

- entre 70 et 90% par les galactomannanes, et
- entre 10 et 30% par les galactanes sulfatés réticulés.

Préférentiellement les galactomannanes et les galactanes sulfatés réticulés forment ensemble un réseau interpénétré.

Plusieurs agents, sous forme de solutions, ont été testés à 7% sur le modèle de peau synthétique et à 0.5% dans l'étude tenseur sensorielle.

Les résultats sont présentés dans les tableaux ci-dessous :

- les combinaisons avec 80% de galactomannanes et 20% de galactanes sulfatés réticulés dans le Tableau 3a
- les combinaisons avec des galactomannanes et de galactanes sulfatés réticulés de masse molaire moyenne sélectionnées selon l'invention dans le Tableau 3b

*Tableau 3a*

| Masses molaires mini-maxi | 20% de Galactanes Sulfatés réticulés de kappaphycus alvarezi | 80% de Galactomannanes de Caesalpinia spinosa | Etat physique | Effet tenseur sur modèle de rétractation | Effettenseur sur panel sensoriel | Effet filmogène au texturomètre (g) |
|---|---|---|---|---|---|---|
| | < 3.24kDa | 4.7kDa | liquide | - | | |
| | < 3.24kDa | 19kDa | liquide | ++ | | |
| 0.7 à 70kDa | 7.2kDa | 10kDa | liquide | ++ | | |
| | 7.2kDa | 16kDa | liquide | ++++ | | |
| | 7.9kDa | 11.7kDa | liquide | ++++ | | |
| 0.9 à 117kDa | 9.4kDa | 13.3kDa | liquide | ++++ | | |
| | 12.8kDa | 20.7kDa | liquide | ++++ | | |
| 1.2 à 171kDa | 14.4kDa | 19kDa | liquide | ++++ | 5.1 | 371 |
| 0.9 à 117 kDa | 14.8kDa | 13.5kDa | liquide | ++++ | | |
| 1.5 à 190kDa | 16.4kDa | 20.2kDa | peu visqueux | ++++ | | |
| NT** : non testé | | | | | | |

Ces résultats montrent que la sélection des galactanes sulfatés réticulés selon leur masse molaire moyenne (5 à 25kDa, préférentiellement 8 à 20kDa) et la sélection des galactomannanes selon leur masse molaire moyenne (5 à 30kDa, préférentiellement 8 à 25kDa) sont nécessaires pour obtenir l'effet attendu.

La combinaison galactomannanes/galactanes sulfatés réticulés dans lesquels soit les galactanes sulfatés réticulés, soit les galactomannanes, soit les deux présentent des masses molaires moyennes en dehors de la sélection de l'invention, ne permettent pas d'obtenir l'état physique, l'effet tenseur et filmogène attendus.

On constate que l'association de galactanes sulfatés sélectionnés réticulés et de galactomannanes sélectionnés selon l'invention permet non seulement de conserver l'effet tenseur de rétractation des galactanes sulfatés sélectionnés réticulés ou des galactomannanes sélectionnés, mais surtout renforcer l'efficacité tenseur perçue par le panel d'experts sensoriels.

*Tableau 3b*

| Galactanes Sulfatés Sélectionnés (GSS) de kappaphycus alvarezi (8-20kDa) | Galactomannanes Sélectionnés (GmS) de Caesalpinia spinosa (8-25kDa) | Effet tenseur sur modèle de rétractation | Effet tenseur sur panel sensoriel | Effet filmogène au texturomètre (g) |
|---|---|---|---|---|
| 10% de GSS réticulés | 90% de GmS | +++ | 4.4 | NT** |
| 20% de GSS réticulés | 80% de GmS | ++++ | 5.1 | 371 |
| 30% de GSS réticulés | 70% de GmS | +++ | 3.8 | NT** |
| 50% de GSS réticulés | 50% de GmS | ++ | NT** | NT** |
| 20% de GSS réticulés | - | +++ | 4.1 | NT** |
| | 80% de GmS | +++ | 4.3 | NT** |
| NT** : non testé | | | | |

Ces résultats montrent l'effet surprenant de l'interaction des deux polysaccharides de tailles sélectionnées. Par exemple pour la combinaison 20% / 80%, l'effet tenseur sur le modèle de rétractation est potentialisé dans la combinaison. Un effet similaire est observé par le panel sensoriel évaluant l'effet tenseur.

Ainsi, les combinaisons contenant entre 10 et 40% de galactanes sulfatés réticulés avec entre 90% et 60% de galactomannanes présentent l'efficacité attendue, un effet tenseur et filmogène.

Par contre, on constate que la combinaison 50%/50% de deux composés de tailles sélectionnées ne présente pas un effet tenseur sur le modèle de rétractation aussi potentialisé que les autres combinaisons selon l'invention.

Cette association permet aussi d'augmenter la résistance du film par rapport à la résistance des films des galactomannanes sélectionnés ou galactanes sulfatés sélectionnés seuls.

L'association des galactanes sulfatés sélectionnés réticulés et des galactomannanes sélectionnés forme en effet un réseau interpénétré.

La présence de ce réseau est démontrée par la mise en évidence de la présence d'un mélange homogène par mesure sur AFM (Microscope de Force Atomique) et de la modification de la mobilité des chaines polymères constitutives du réseau par des études de DSC (Calorimétrie Différentielle à balayage) et de DMA (Analyse Mécanique Dynamique).

Le principe de fonctionnement de l'AFM consiste à sonder la surface d'un échantillon par balayage successif d'une sonde très fine située au voisinage immédiat de cette surface. La sonde AFM est constituée d'un levier flexible à l'extrémité duquel est fixée une pointe. L'échantillon est fixé sur une céramique piézoélectrique. Cette céramique permet des déplacements dans les trois directions de l'espace.

Le microscope à force atomique permet de visualiser aussi bien des structures biologiques complexes que des molécules uniques, et cela dans leur état fonctionnel. Les résolutions latérale et verticale peuvent atteindre quelques angströms. En fonction du mode de mesure (contact, tapping, no contact) et de la fonctionnalisation de la sonde, le microscope à force atomique permet de mesurer des forces intra- et intermoléculaires, des affinités entre molécules, de réaliser la microrhéologie ou encore d'évaluer la topologie d'une surface.

Les échantillons analysés par AFM ont été préalablement préparés par spin coating.

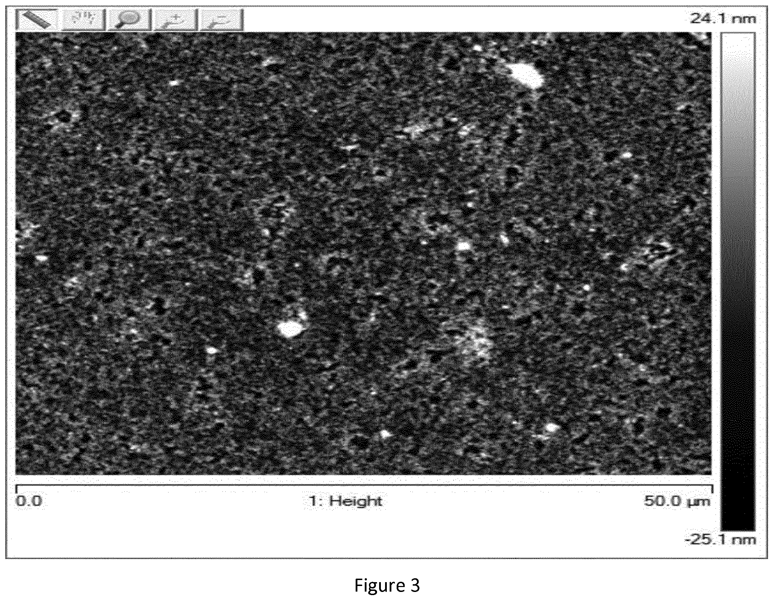

La topologie obtenue par AFM du film de l'agent selon l'invention (galactanes sulfatés réticulés et galactomannanes - ex2) est visualisée à une échelle de $50\mu m$ (Figure 3). L'image AFM de l'association des galactanes sulfatés réticulés et des galactomannanes selon l'invention, montre que le mélange est homogène, il n'y a pas de micro-domaines de chaque polymère. La rugosité de surface de l'agent selon l'invention est de 7nm.

Les changements d'état des polymères sont des processus endothermiques (par exemple, fusion) ou exothermiques (par exemple, cristallisation). Ces échanges thermiques peuvent être mesurés par analyse enthalpique différentielle (DSC). Si le degré d'interpénétration de deux polymères n'est pas important, les deux réseaux seront répartis dans deux phases et dans ce cas, deux températures de transition vitreuse correspondant à chacun des réseaux pris individuellement seront détectés. Si l'on a affaire à un réseau interpénétré, une seule température de transition vitreuse sera détecté à une température intermédiaire entre celles des deux réseaux combinés.

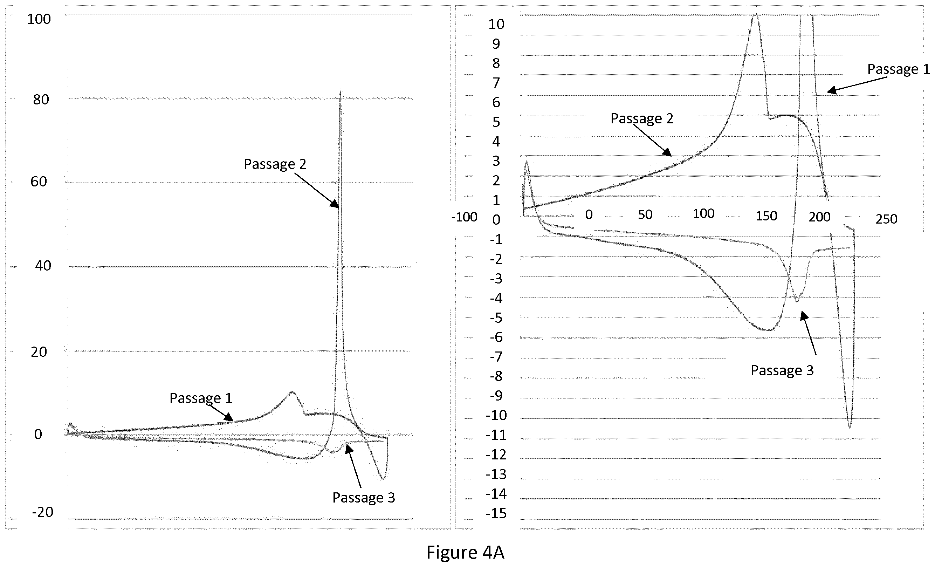

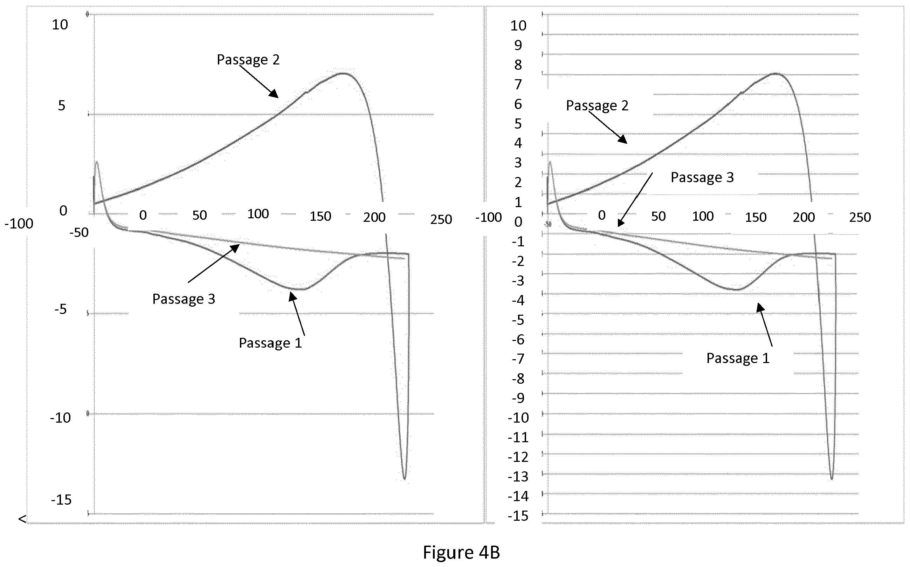

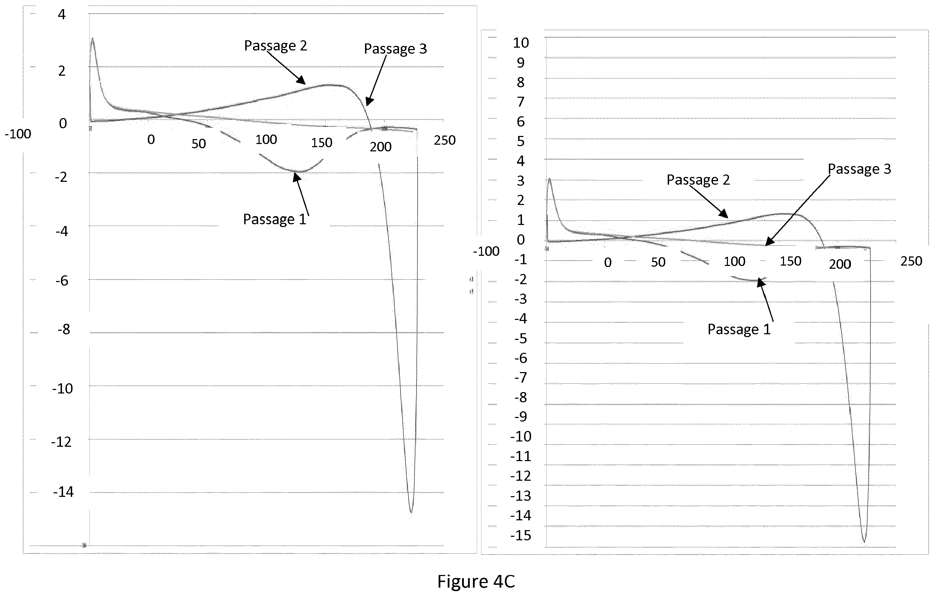

Les échantillons testés sont les galactanes sulfatés réticulés sélectionnés de masse molaire moyenne 10.8kDa (Figure 4A), les galactomannanes sélectionnés de masse molaire moyenne 10kDa (Figure 4B) et l'association de ces galactanes sulfatés sélectionnés réticulés et de ces galactomannanes sélectionnés (Figure 4C).

La comparaison des spectres des biopolymères simples et de l'association (exemples de l'exemple 1) montre un changement dans le comportement thermique de l'association par rapport aux biopolymères simples. En effet, malgré la répartition galactanes sulfatés / galactomannanes, on peut observer l'absence du pic exothermique pour l'association mais présent dans l'échantillon des galactanes sulfatés réticulés. De plus, l'endotherme caractéristique d'une fusion des zones cristallines des galactanes sulfatés réticulés est absente dans l'association suggérant une répartition homogène des deux réseaux galactanes sulfatés réticulés et galactomannanes, limitant l'établissement de zone cristalline imputable aux galactanes sulfatés réticulés.

L'analyse thermomécanique dynamique (DMA) est communément utilisée pour mettre en évidence la présence de différentes phases dans les mélanges de polymères. Lorsqu'il n'y a pas combinaison de deux polymères, on observe deux températures de relaxation mécanique, une pour chaque polymère. Lorsque la combinaison de deux polymères est parfaitement homogène, une seule température de relaxation mécanique est observée. Elle est généralement située entre les températures de relaxation mécanique des deux polymères constitutifs de la combinaison .

La relaxation mécanique est évaluée au moyen d'un paramètre appelé, la tan delta. Si les interactions sont suffisamment importantes dans le mélange de deux polymères, un pic additionnel de tan delta peut être observé.

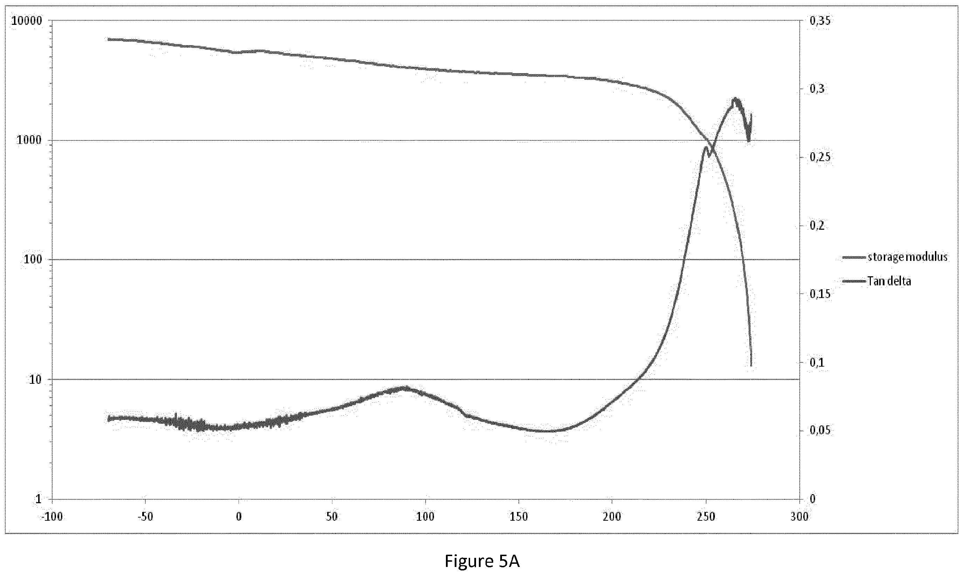

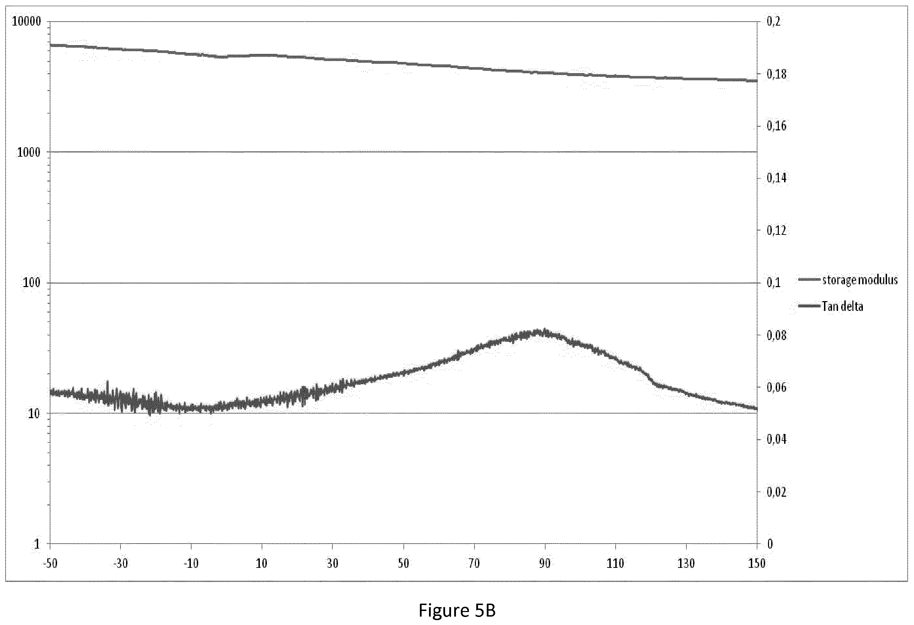

On observe sur la courbe DMA des galactomannanes sélectionnés (-70 à 300°C, Figure 5A et grossissement -50 à 150°C, Figure 5B) la présence d'un pic de tan delta à 90°C.

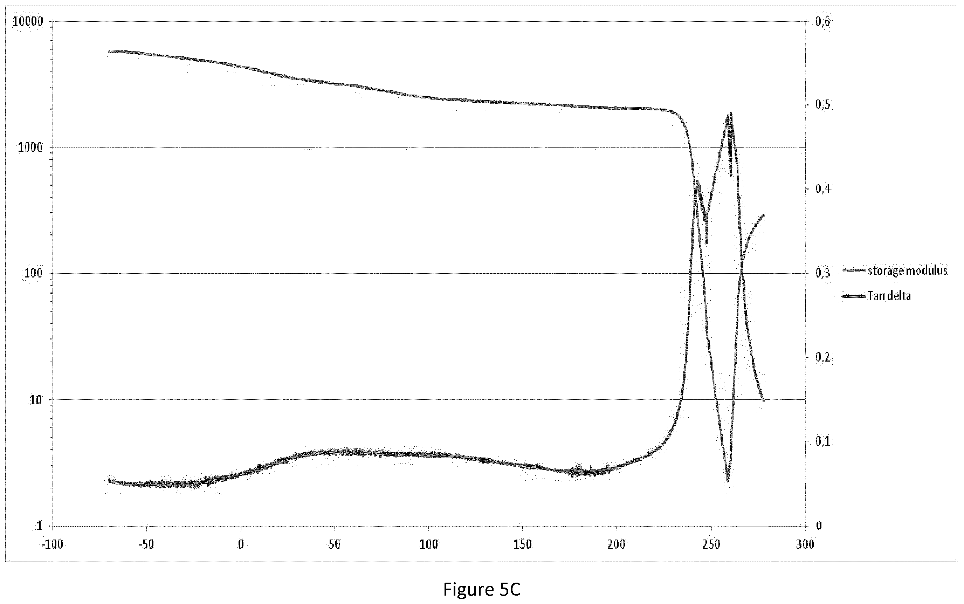

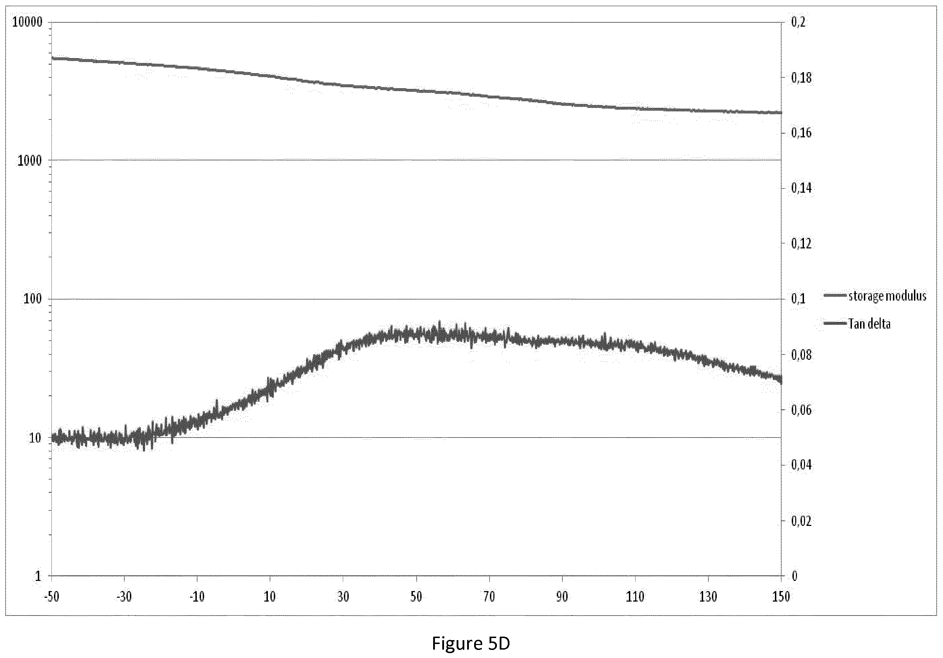

La courbe DMA, de l'agent selon l'invention associant les galactomannanes et les galactanes sulfatés réticulés (-70 à 300°C, Figure 5C et grossissement -50 à 150°C, Figure 5D) présente un profil très élargi vers 40 et 100°C démontrant une plage de température de relaxation mécanique plus étendue que celles des galactomannanes seuls.

Cette modification dans la Tan delta ne peut avoir lieu que parce que les deux réseaux ne présentent pas de séparation de phase et qu'ils sont suffisamment proches l'un de l'autre. Cet élargissement de la Tan delta ne s'accompagnant pas de la présence de deux pics distincts, il suggère la présence de deux réseaux interagissant l'un avec l'autre.

Les modifications des températures de relaxation (DMA) et de la transition vitreuse (DSC) de l'agent selon l'invention démontre une répartition homogène des deux réseaux des biopolymères. Elles suggèrent également la mise en place d'interaction entre ces deux biopolymères relatif à un réseau interpénétré de biopolymères.

L'agent cosmétique ou dermocosmétique selon l'invention peut donc être utilisé pour ses différentes propriétés. En particulier, l'invention vise son utilisation comme agent cosmétique ou dermocosmétique tenseur et/ou filmogène.

Il peut ainsi être utilisé en particulier comme agent :

- pour un effet cosmétique ou dermocosmétique protecteur de la peau contre la pénétration de molécules toxiques, telles que les polluants, les allergènes, les irritants ou les métaux lourds et/ou l'adhésion bactérienne de Staphylococcus aureus, et/ou pour améliorer l'effet barrière de la peau, et/ou
- pour un effet cosmétique ou dermocosmétique film liftant, pour améliorer l'éclat de la peau et/ou lisser la peau, et/ou
- pour un effet cosmétique film perceptible, afin de sentir l'efficacité tenseur, améliorer l'apparence globale du visage et favoriser la tenue des pigments du maquillage et/ou
- pour un effet film seconde peau, booster de beauté.

Du fait de ces différentes efficacités, l'invention vise aussi son utilisation pour lutter contre les manifestations disgracieuses du vieillissement de la peau.

L'invention a donc également pour objet :

- l'utilisation cosmétique non thérapeutique d'un agent selon l'invention :

    * comme agent cosmétique tenseur et/ou filmogène, et/ou
    * comme agent cosmétique pour améliorer l'effet barrière de la peau, et/ou
    * pour améliorer l'éclat de la peau et/ou lisser le microrelief de la peau et/ou lisser les rides, et/ou
    * pour lutter contre les manifestations disgracieuses du vieillissement de la peau, et/ou
    * pour favoriser la tenue du maquillage et/ou améliorer la dispersion des pigments dans les formulations cosmétiques, et/ou
    * pour un effet seconde peau, booster de beauté.

- l'agent cosmétique selon l'invention pour une utilisation :

    * dans la protection de la peau,
    * dans la protection de la peau contre la pénétration de molécules toxiques,
    * dans la protection de la peau contre l'adhésion bactérienne de pathogènes.

L'agent cosmétique et/ou dermocosmétique selon l'invention est préférentiellement utilisé dans une composition, cette composition comprenant un milieu cosmétiquement acceptable. Il s'agit de compositions dans différentes formes galéniques, adaptées à l'administration par voie topique sur la peau humaine.

Ces compositions peuvent se présenter notamment sous forme d'émulsions huile-dans-eau, émulsions eau-dans-huile, émulsions multiples (Eau/Huile/Eau ou Huile/Eau/Huile) qui peuvent être éventuellement des microémulsions ou des nanoémulsions, ou sous forme de solutions, suspensions, hydrodispersions, gels aqueux ou poudres. Elles peuvent être plus ou moins fluides et avoir l'aspect d'une crème, d'une lotion, d'un lait, d'un sérum, d'une pommade, d'un gel, d'une pâte ou d'une mousse, ou sous forme solide.

Il peut s'agir de compositions comprenant au moins 0.01% d'un agent selon l'invention, préférentiellement entre 0.05 et 0.5%.

Ces compositions comprennent, outre l'actif, un milieu physiologiquement acceptable et de préférence cosmétiquement acceptable, c'est-à-dire qui ne provoque pas de sensations d'inconfort inacceptables pour l'utilisateur telles que des rougeurs, tiraillements ou picotements.

Les compositions selon l'invention peuvent contenir comme adjuvant au moins un composé choisi parmi :

- les huiles, qui peuvent être choisies notamment parmi les huiles de silicone, linéaires ou cycliques, volatiles ou non volatiles ;
- les cires, telles que l'ozokérite, la cire de polyéthylène, la cire d'abeille ou la cire de carnauba,
- les élastomères de silicone,
- les tensioactifs, de préférence émulsionnants, qu'ils soient non ioniques, anioniques, cationiques ou amphotères,
- les co-tensioactifs, tels que les alcools gras linéaires,
- les épaississants et/ou gélifiants,
- les humectants, tels que les polyols comme la glycérine,
- les filtres organiques,
- les filtres inorganiques,
- les colorants, les conservateurs, les charges, les pigments, les minéraux,
- les tenseurs,
- les séquestrants,
- les parfums,
- et leurs mélanges, sans que cette liste soit limitative.

Des exemples de tels adjuvants sont cités notamment dans le Dictionnaire CTFA *(International Cosmetic Ingrédient Dictionary and Handbook* publié par le *Personal Care Product Council).*

Bien entendu, l'homme du métier veillera à choisir les éventuels composés complémentaires, actifs ou non-actifs, et leur quantité, de telle sorte que les propriétés avantageuses du mélange ne soient pas, ou sensiblement pas, altérées par l'adjonction envisagée.

Ces compositions sont notamment destinées à être utilisées pour les effets procurés par l'agent cosmétique ou dermocosmétique selon l'invention.

L'invention vise aussi spécifiquement un procédé cosmétique de soin de la peau pour améliorer l'état de la peau, en particulier pour améliorer l'éclat de la peau et/ou lutter contre les manifestations disgracieuses du vieillissement de la peau. Préférentiellement le procédé consiste à appliquer au moins une fois par jour sur la peau du visage une composition comprenant au moins 0,05% en poids de matières sèches de l'agent cosmétique ou dermocosmétique selon l'invention.

Afin d'illustrer l'invention, des exemples et des résultats d'essais sont présentés en suivant.

EXEMPLES

Exemple 1 : Exemples d'agents selon l'invention

Plusieurs agents (présentés dans le Tableau 4) selon l'invention ont été testés dans les essais suivants. les résultats donnés pour les essais sur l'évaluation de l'effet de l'agent selon l'invention, représentent la moyenne des résultats individuels de certains de ces agents.

***Tableau 4***

| Essai | Produits B Galactanes Sulfatés réticulés de kappaphycus alvarezi | | Produits A Galactomannanes de Caesalpinia spinosa | | Effet tenseur sur modèle de rétractation |
|---|---|---|---|---|---|
| | Teneur | Masse molaire moyenne | Teneur | Masse molaire moyenne | |
| 1 | 20% | 7.2kDa | 80% | 16kDa | ++++ |
| 2 | 20% | 7.9kDa | 80% | 11.7kDa | ++++ |
| 3 | 20% | 9.4kDa | 80% | 13.3kDa | ++++ |
| 4 | 20% | 12.8kDa | 80% | 20.7kDa | ++++ |
| 5 | 20% | 14.4kDa | 80% | 19kDa | ++++ |
| 6 | 20% | 14.8kDa | 80% | 13.5kDa | ++++ |
| 7 | 20% | 16.4kDa | 80% | 20.2kDa | ++++ |
| 8 | 10% | 14.4kDa | 90% | 19kDa | +++ |
| 9 | 30% | 14.4kDa | 70% | 19kDa | +++ |

L'homme de métier sait modifier le temps d'hydrolyse et/ou la température d'hydrolyse pour obtenir des biopolymères de type galactanes sulfatés ou des galactomannanes de masses molaires moyennes attendues. Ces agents sont obtenus selon le procédé comprenant les étapes suivantes :

- Préparation du produit A :

  ◦ Solubilisation de poudre de galactomannanes natifs de Caesalpinia spinosa dans l'eau à 20g/l,

  ◦ Hydrolyse enzymatique ménagée pendant 20mn à 6h, à une température entre 20 et 60°C,

  ◦ Filtration afin de séparer les phases soluble et insoluble, et éliminer la phase insoluble,

- Préparation du produit B :

  ◦ Solubilisation de poudre de galactanes sulfatés natifs de *Kappaphycus alvarezii* dans l'eau à 20g/l,

  ◦ Hydrolyse acide ménagée pendant 10mn à 6h, à une température entre 20 et 60°C,

  ◦ Filtration afin de séparer les phases soluble et insoluble, et éliminer la phase insoluble,

- Mélange du produit A et du produit B.

- Ajout de l'agent de réticulation,

- Filtration membranaire et sélection des polysaccharides de masses molaires entre 1 et 150kDa.

Exemple 2 : Exemple de composition selon l'invention de type émulsion

L'émulsion a été réalisée en utilisant la formule suivante :

| | |
|---|---|
| Myristyl alcohol / Myristyl glucoside (Montanov 14 - SEPPIC) | 5,00% |
| Isopropylpalmitate (DUB IPP - Stéarinerie DUBOIS) | 25,00% |
| Amyl cinnamaldéhyde | 0,20% |
| AGENT SELON L'INVENTION | 1,00% |
| Kathon CG (Rhom & Haas) | 0,05% |
| Eau | qsp 100% |

### Exemple 3 : exemple de composition selon l'invention de type gel-émulsionné

Le gel-émulsionné a été réalisé en utilisant la formule suivante :

| | |
|---|---|
| Cetearyl ethylhexanoate (Lanol 1688, Seppic) | 10,00 % |
| Behenyl alcohol / Arachidyl glucoside / Arachidyl alcohol (Montanov 202, Seppic) | 3,00% |
| Polyacrylamide / C13-14 isoparaffin / Laureth-7 (Sepigel 305, Seppic) | 2,00% |
| Isonyl isononanoate (Lanol 99, Seppic) | 2,00% |
| Conservateurs | 0,70% |
| Agent selon l'invention | 0,05%, 0,10%, 0,25% ou 0,50% |
| Eau | qsp 100% |

### Exemple 4 : exemple de composition selon l'invention de type gel

Le gel a été réalisé en utilisant la formule suivante :

| | |
|---|---|
| Agent selon l'invention | 0,05%, 0,10%, 0,25% ou 0,50% |
| Conservateur | 0,23% |
| Carbomer (Ultrez 10, Noveon) | 0,27% |
| Eau | qsp 100% |

### Exemple 5 : exemple de composition selon l'invention de type sérum

Le sérum a été réalisé en utilisant la formule suivante :

| | |
|---|---|
| Glycerin | 3,0 % |
| Propylene glycol | 2,0 % |
| Dimethicone (DC200, Dow Corning) | 2,0 % |
| Caprilic/capric triglyceride (DUB, MCT5545, Stéarinerie Dubois) | 2,0 % |
| PEG-7 glyceryl cocoate (DUB CG7, Stéarinerie Dubois) | 1,4 % |
| Agent selon l'invention | 1,0% |
| Conservateurs | 1,0% |
| Cyclopentasiloxane / cyclohexasiloxane (Xiameter PMX-0345, Dow Corning) | 1,0% |
| Acrylates/C10-30 Alkyl acrylate crosspolymer (Carbopol Ultrez 20, Lubrizol) | 0,4% |
| Xanthan gum (Keltrol CG, Kelco) | 0,4% |
| Carrageenan (Satiagel™ UTH 18, Cargill) | 0,2% |
| Eau | qsp 100% |

### Exemple 6 : exemple de composition selon l'invention de type fond de teint

Le fond de teint a été réalisé en utilisant la formule suivante :

| | |
|---|---|
| Cetyl PEG / PPG-10 / 1 Dimethicone (E1016, THOR) | 24,0% |
| PEG-12 Dimethicone (E1200, THOR) | 16,0% |
| Cyclopentasiloxane / Cyclohexasiloxane (XIAMETER PMX-0345, XIAMETER) | 10,0% |
| Methyl Trimethicone / Acrylate / Dimethicone Copolymer (KP - 549, SHIN ETSU) | 8,0% |
| Titanium Dioxyde (C.I. 77891) (SunPuro™ TitaniumDioxyde, SUN CHEMICAL) | 7,4% |
| Heptyl glucoside (SEPICLEAR G7, SEPPIC) | 7,0% |
| Polydimethylsiloxyethyl Dimethicone (K- 6028, SHIN ETSU) | 5,0% |
| Dimethicone / Trisiloxane / Ceteth-10 / Laureth-4 (DC 7-3110, DOWN CORNING) | 5,0% |
| Myristyl alcohol / Myristyl glucoside (MONTANOV 14, SEPPIC) | 4,0% |
| Octyldodecanol / Octydodecyl Xyloside / PEG-30 Dipolyhydroxystearate (EASYNOV, SEPPIC) | 3,0% |
| Methyl trimeticone (TMF 1,5, SHIN ETSU) | 2,0% |

(suite)

| | |
|---|---|
| Zinc oxyde (Oxyde de Zinc, DEGUSSA) | 2,0% |
| Titanium Dioxyde / Butylene Glycol Dicaprylate/Dicaprate / Silica / Polyglyceryl-2 Dipolyhydroxystearate (EUSOLEX T OLEO, MERCK) | 2,0% |

| | |
|---|---|
| Iron Oxides (C.I. 77492) (SunPuro™ Yellow Iron Oxide, SUN CHEMICAL) | 1,3% |
| Iron Oxides (C.I. 77499) (SunPuro™ Black Iron Oxide, SUN CHEMICAL) | 1,2% |
| Cetearyl Alcohol / Cetearyl Glucoside (MONTANOV 68, SEPPIC) | 1,0% |
| Talc (HYTECH) | 1,0% |
| Conservateurs | 0,7% |
| *Oriza sativa* (rice) starch (Poudre de riz, HYTECH) | 0,7% |
| Agent selon l'invention | 0,5% |
| Iron Oxides (C.I. 77491) (SunPuro™ Red Iron Oxide, SUN CHEMICAL) | 0,4% |
| Candelilla cera (Cire de candelilla, HYTECH) | 0,3% |
| Mica, Iron oxide (CI 77019, CI 77491) (Mica, HYTECH) | 0,3% |
| Eau | qsp 100% |

Exemple 7 : exemple de omposition de type masque-crème

Le masque-crème a été réalisé en utilisant la formule suivante :

| | |
|---|---|
| Isonyl isononanoate (Lanol 99, Seppic) | 10,0 % |
| Cetearyl ethylhexanoate (Lanol 1688, Seppic) | 5,0 % |
| Behenyl alcohol / Arachidyl glucoside / Arachidyl alcohol (Montanov 202, Seppic) | 5,0 % |
| Cetearyl alcohol / Cetearyl glucoside (Montanov 68, Seppic) | 5,0 % |
| Propylene glycol | 3,5% |
| Conservateurs | 0,7% |
| Agent selon l'invention | 0,5% |
| Sodium alginate (Satialgine S550, Cargill) | 0,1% |
| Eau | qsp 100% |

Exemple 8 : exemple de composition de type masque tissu

La solution pour imbiber le masque tissu est la suivante :

| | |
|---|---|
| MethylPropanediol (DUB DIOL, Stéarinerie Dubois) | 7,0% |
| Glycerol | 1,5% |
| Butylen glycol | 1,0% |
| Conservateurs | 1,0% |
| Agent selon l'invention | 0,5% |
| PEG-7 glyceryl cocoate (DUB CG7, Stéarinerie Dubois) | 0,2% |
| Carbomer (CARBOPOL U20, Lubrizol) | 0,2% |
| Sodium Dilaureth-7 Citrate (EUCAROL D , CESALPINIA) | 0,1% |
| Eau | qsp 100% |

ESSAIS - DEMONSTRATION DE L'EFFICACITE DE L'AGENT SELON L'INVENTION

Les performances de l'agent selon l'invention ont été évaluées selon 4 axes :
Il présente un effet film protecteur, un effet film liftant, un effet film perceptible et un effet film seconde peau booster de beauté.

1) Effet film protecteur :

L'agent selon l'invention présente un maillage dense qui lui confère des effets protecteurs sur la peau. Cet effet film forme à la surface de l'épiderme une barrière moléculaire capable de protéger des agressions extérieures (chimiques ou mécaniques) tout en maintenant les échanges hydriques de la peau.

Des études réalisées *ex-vivo* ou *in vivo* ont permis d'établir que l'agent selon l'invention réduit la pénétration des polluants dont les particules fines de type PM10 et les particules de carbone. Il exerce des effets similaires sur la pénétration des allergènes, des irritants et des métaux lourds. Il permet aussi de limiter l'adhésion bactérienne de *Staphylococcus* aureus sur la peau. L'ensemble de ces effets est rapide puisque l'effet bouclier est observable dès 15 minutes et jusqu'à 24 heures après application.

De plus, le biopolymère de l'invention protège la fonction barrière de la peau soumise à des agressions mécaniques. Un test réalisé *in vivo* démontre que l'application biquotidienne d'un agent selon l'invention pendant 21 jours permet la réduction des pertes insensibles en eau (PIE) induites par un stress mécanique (= strippings).

Une analyse complémentaire a également permis de démontrer l'action non-occlusive de l'agent selon l'invention : en l'absence d'agression il ne modifie pas les pertes insensibles en eau de la peau.

a) Effet de l'agent selon l'invention sur la pénétration des polluants

Les particules fines émises au cours d'épisodes de pollution entraînent des dommages cutanés et sont à l'origine de l'apparition de rides ou de tâches pigmentaires. L'objectif de cette étude est d'évaluer in vivo l'effet protecteur d'un agent selon l'invention vis-à-vis de l'adhésion de particules fines de pollution de l'air d'un diamètre de $10\mu m$ sur la peau. Les particules fines sont constituées de différents polluants comme des hydrocarbures polyaromatiques (PAH), et leutres formes nitrés (nitro-PAH), des produits chimiques chlorés (PCB), des pesticides, des hydrocarbures, des métaux lourds. Elles sont collectées dans des zones urbaines.

Cette étude a été réalisée sur 10 volontaires présentant une peau saine au niveau de la face interne des avant-bras et des explants de peaux préalablement altéré afin de mimer une peau humaine altérée.

L'altération des explants est réalisée par stripping avec de la colle cyanoacrylate. Cette méthode physique permet de décoller la partie superficielle de l'épiderme, diminuant ainsi la fonction barrière de la peau. L'altération est validée par mesure de la résistance électrique transépithéliale (TEER) qui doit être réduite de 80%.

Les particules fines ont été quantifiées sur photographies avant et après applications des particules fines, ainsi qu'après leur rinçage.

L'agent selon l'invention en solution aqueuse à différentes concentrations (0,10%, 0,25%, 0,50% et 1,00%) est appliqué à la surface de la peau sur $2cm^2$. Après 20mn, les zones cutanées sont photographiées, et une suspension de particules fines est appliquée. Après 50mn, de nouvelles photographies des zones sont réalisées avant et après un rinçage standardisé.

La quantité des particules ayant adhéré sur la surface cutanée est proportionnelle à la couleur de la zone. Plus une zone est claire, moins les particules ont adhérés.

Les moyennes des résultats sur le panel de 10 volontaires sont présentés dans le Tableau 5a et sur les 16 explants de peaux altérées dans le Tableau 5b.

***Tableau 5a***

|  | Adhésion des particules après rinçage (%) | % adhésion des particules / Témoin |
|---|---|---|
| Eau distillée | 68 | |
| Agent à 0.1% | 52 | -23% |
| Agent à 0.25% | 49 | -28% |
| Agent à 0.5% | 42 | -38% |
| Agent à 1.0% | 36 | -47% |

Dans les conditions de cette étude, en formant un effet film protecteur sur la surface de la peau, l'agent cosmétique ou dermocosmétique selon l'invention à 1% réduit de 47% l'adhésion des particules fines sur la peau.

### Tableau 5b

| | Adhésion des particules après rinçage (%) | % adhésion des particules / Témoin |
|---|---|---|
| Eau distillée | 64 | |
| Agent à 0.1% | 53 | -17% |
| Agent à 0.25% | 50 | -22% |
| Agent à 0.5% | 47 | -26% |
| Agent à 1.0% | 37 | -42% |

Dans les conditions de cette étude, en formant un effet film protecteur sur la surface de la peau altérée, l'agent cosmétique ou dermocosmétique selon l'invention à 1% réduit de 42% l'adhésion des particules fines sur la peau altérée. Donc l'agent cosmétique ou dermocosmétique selon l'invention protège la peau des effets délétères de la pollution.

b) Effet de l'agent selon l'invention sur la pénétration des irritants

L'objectif de cette étude a été de démontrer que l'agent selon l'invention empêche la pénétration d'un irritant cutané. L'effet protecteur est évalué pour l'agent formulé à 0,50% en gel-émulsionné (composition de l'exemple 3) dans le cadre d'un "stinging test". Ce test permet de déterminer la capacité des panélistes à percevoir et à évaluer les sensations engendrées par l'application d'une solution d'acide lactique au niveau des ailes du nez. En effet, l'application de cette solution irritante entraîne l'apparition de sensations inconfortables telles que picotements, brûlures, démangeaisons.

L'étude a été réalisée sur 12 volontaires sains. L'effet des gels émulsionnés testés sur la réactivité cutanée après application d'une solution d'acide lactique a été évalué sur une échelle numérique en 4 points avant et après application.

Dans les conditions de cette étude, l'agent selon l'invention formulé à 0,50% diminue significativement de 22% les sensations inconfortables engendrées par l'application d'un agent irritant. Cet effet a été perçu par 58% des volontaires.

En formant un effet film protecteur, l'agent selon l'invention préserve les peaux sensibles vis-à-vis des irritants.

c) Effet de l'agent selon l'invention sur la pénétration des allergènes

L'objectif de cette étude a été d'évaluer la capacité de l'agent selon l'invention à empêcher la pénétration transcutanée d'un allergène en formant un film protecteur à la surface de la peau. L'étude a été réalisée sur explants de peau en utilisant les cellules de diffusion de Frantz en fonctionnement statique.

L'effet barrière de l'agent selon l'invention a été évalué en quantifiant la capacité d'un allergène (amyl cinnamaldéhyde) formulé à 0.2% dans une émulsion (composition de l'exemple 4), à traverser la barrière cutanée *ex vivo* en présence ou non de l'agent selon l'invention. Le taux d'amyl cinnamaldéhyde est quantifié par chromatographie en phase gazeuse couplée à la spectrométrie de masse.

Le pourcentage d'allergène présent dans la peau est calculée à l'aide de la formule suivante :

$$\% \text{ allergène dans la peau} = 100 - \left( \frac{QM_{T24}}{QM_{ini}} \right) \times 100$$

Avec :

$QM_{T24}$ : quantité d'allergène dans la formule récupérée à la surface de la peau à t = 24 heures.
$QM_{ini}$ : quantité d'allergène dans la formule déposée à la surface de la peau.

Les quantités d'allergène présent dans la peau après 24 heures sont données dans le Tableau 6.

**Tableau 6**

|  | % d'allergène dans la peau | Variation par rapport au témoin |
|---|---|---|
| Témoin | 33,9 | |
| Agent à 1,00% | 14,4 | - 62% |

Dans les conditions de l'étude, en formant un film protecteur à la surface de la peau, l'agent selon l'invention à 1,00%, réduit la pénétration transcutanée de l'amyl cinnamaldéhyde de 62%.

d) Effet de l'agent selon l'invention sur la pénétration des métaux lourds

L'objectif de cette étude est d'évaluer ex vivo la capacité de l'agent selon l'invention à empêcher la pénétration transcutanée des métaux lourds en formant un film protecteur à la surface de la peau. L'étude a été réalisée après dépôt du cadmium à la surface de la peau en utilisant des cellules de diffusion de Frantz en fonctionnement statique. Le taux de métaux lourds ayant pénétré a été quantifié par spectrométrie d'émission optique à source plasma (ICP-OES).
L'agent selon l'invention a été déposé à 0.5% à la surface de la peau. Après plusieurs heures, un dépôt de 4ppm de cadmium est réalisé sur les explants. Après 14h, le cadmium à la surface de l'explant est récupéré à l'aide d'un strip. Le cadmium contenu dans les différents échantillons est extrait dans une solution d'acide nitrique. Tous ces échantillons sont dosés en ICP-OES. Les résultats sont présentés dans le Tableau 7.

**Tableau 7**

|  | % de cadmium dans la peau | Variation par rapport au témoin |
|---|---|---|
| Témoin | 69.1 | |
| Agent à 1,00% | 40.7 | - 41% |

Dans les conditions de l'étude, l'agent selon l'invention à 0.5% réduit la pénétration transcutanée du cadmium de 41% dans une peau saine. En formant un film protecteur à la surface de la peau, il la protège contre les effets délétères des métaux lourds présents dans la pollution.

e) Effet de l'agent selon l'invention sur l'adhésion bactérienne de *Staphylococcus* aureus sur la peau

L'objectif de cette étude est d'évaluer in vitro la capacité de l'agent selon l'invention à empêcher la formation d'un biofilm par la bactérie Staphylococcus aureus (SA). Cette bactérie est une bactérie pathogène présente en quantité anormalement élevée dans divers désordres cutanés. En adhérant à la surface de la peau, elle forme un biofilm qui est à l'origine d'une perturbation de l'homéostasie de la peau.
Cette étude est réalisée sur des épidermes reconstruits après application d'une solution de Staphylococcus aureus. L'adhésion et la colonisation par cette bactérie est visualisée par microscopie électronique à balayage (MEB).
Les épidermes reconstruits sont traités topiquement avec 0.5% de l'agent selon l'invention. Après 24h, ils sont traités topiquement avec une solution de SA et incubés pendant 24h. La visualisation du biofilm est ensuite réalisée.

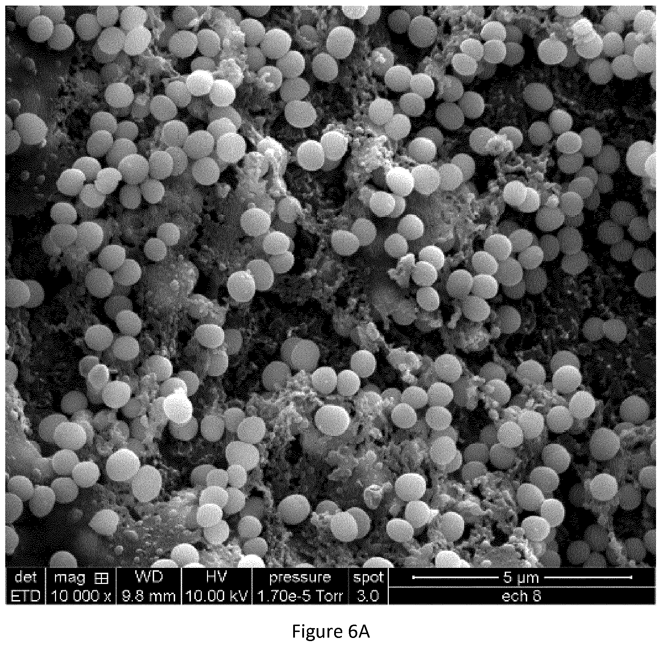

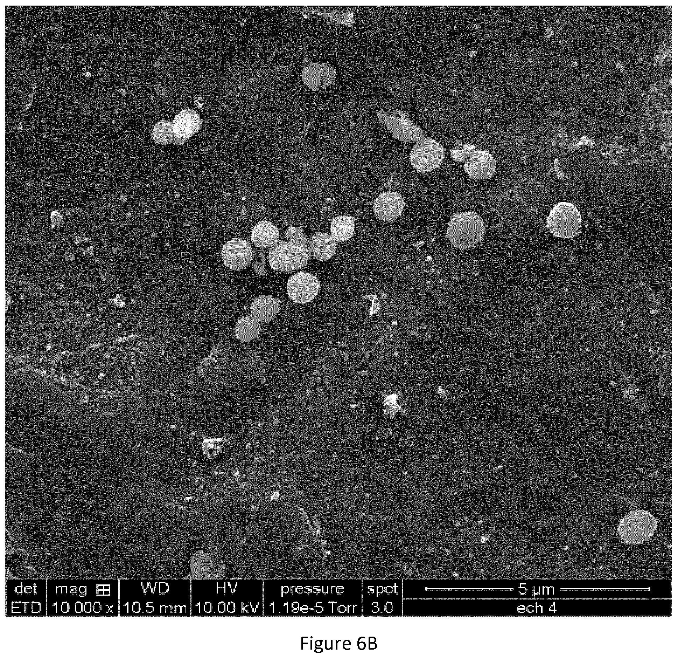

La Figure 6A représente le biofilm de SA sur l'épiderme reconstruit témoin, la Figure 6B sur l'épiderme reconstruit traité par l'agent selon l'invention.
Testé à 0.5% sur épidermes reconstruits, l'agent selon l'invention limite l'adhésion de Staphylococcus aureus et consécutivement la formation du biofilm.

f) Effet de l'agent selon l'invention sur la barrière cutanée

L'objectif de l'étude a été d'évaluer, in vivo, la capacité de l'agent selon l'invention formulé à 0,50% en gel-émulsionné (composition telle que présentée dans la partie exemples), à préserver la fonction barrière soumise à une agression mécanique (5 strippings).
Cette étude a été réalisée sur 11 volontaires sains. Les pertes insensibles en eau ont été mesurées à l'aide du Tewamètre®TM300 (Courage et Khazaka).
Les résultats sont présentés dans le Tableau 8.

**Tableau 8**

| | PIE (g/h/m$^2$) | | |
|---|---|---|---|
| | Avant agression | Après agression | Variation / Placebo (%) |
| Placebo | 5,8 | 7,6 | |
| Agent à 0,50% | 5,9 | 6,9 | -14 |

Dans les conditions de cette étude, en comparaison au placebo, l'agent selon l'invention formulé à 0,50% limite significativement de 14% les pertes insensibles en eau engendrées par une agression mécanique.

En formant un effet film protecteur, l'agent selon l'invention permet de renforcer la barrière cutanée vis-à-vis d'une agression mécanique

**2) Effet film liftant:**

De nombreuses analyses ont été menées *in vivo* démontrant la plurifonctionnalité de l'agent selon l'invention. Il apparait comme un puissant agent liftant capable de :

- lisser la peau au niveau de deux zones du corps (avant-bras et ventre) ;
- améliorer les propriétés biomécaniques de la peau ;
- réduire les rides de la patte d'oie et du contour des lèvres.

Ces études ont permis de mesurer l'influence de divers paramètres : la dose de l'agent, la durée du traitement et l'impact de la formulation. L'efficacité a été quantifiée par voie instrumentale ou évaluée par les volontaires eux-mêmes.

Ainsi, il a été montré qu'à court terme, l'agent selon l'invention présente un effet tenseur fort et dose-dépendant. Cet effet visible dès 30 minutes reste significatif après deux heures. Il permet le lissage de la peau au niveau de l'avant bras et du ventre et aussi la réduction immédiate des rides au niveau de la patte d'oie ou autour des lèvres.

A long terme : l'agent selon l'invention démontre son efficacité antirides puisqu'il permet après 7 jours de cure de combler visiblement les rides du contour des lèvres. L'effet lissant au niveau du ventre se maintient jusqu'à 14 jours.

Ainsi, l'agent selon l'invention présente des propriétés liftantes. Il permet une amélioration du microrelief cutané au niveau de différentes zones du corps et le gommage des rides du visage. Il peut donc être intégré dans les soins liftant et anti-âge.

a) Effet lissant de l'agent selon l'invention sur le microrelief cutané

L'effet lissant de l'agent selon l'invention sur le microrelief cutané est évalué sur deux zones : les avant-bras et le ventre.

- L'objectif de la première étude a été d'évaluer, *in vivo,* contre placebo l'effet lissant au niveau de l'avant-bras de l'agent selon l'invention, formulé à différentes doses (0,05%, 0,10%, 0,25% et 0,50%) dans un gel-émulsionné (composition de l'exemple 3), 30 minutes et 2 heures après une application isolée.
- L'objectif de la seconde étude a été d'évaluer, *in vivo,* contre placebo l'effet lissant de l'agent selon l'invention, formulé à 0,10% en gel-émulsionné (composition de l'exemple 3) au niveau du ventre après 1heure et 14 jours d'applications biquotidiennes. L'effet lissant a été mesuré par projection de franges (système Eotech) après réalisation d'empreintes. Les paramètres caractéristiques de la rugosité du microrelief de la peau (Sa et Sq) sont évalués.

Les résultats sont présentés dans les Tableaux 9 et 10.

| | Variation / Placebo (%) | | | |
|---|---|---|---|---|
| | 30 minutes | | 2 heures | |
| | Paramètre Sa | Paramètre Sq | Paramètre Sa | Paramètre Sq |
| Agent à 0,05% | -4,1% | -4,1% | -4,0% | -4,4% |
| Agent à 0,10% | -4,8% | -4,5% | -4,7% | -4,7% |
| Agent à 0,25% | -6,1% | -5,4% | -5,0% | -4,9% |
| Agent à 0,50% | -6,8% | -6,7% | -5,4% | -5,5% |

*Tableau 9*

| | Variation / Placebo (%) | | | |
|---|---|---|---|---|
| | 1 heure | | 14 jours | |
| | Paramètre Sa | Paramètre Sq | Paramètre Sa | Paramètre Sq |
| Agent à 0,10% | -8,6% | -9,6% | -9,0% | -9,8% |

*Tableau 10*

Dans les conditions de ces études, l'agent selon l'invention présente un effet tenseur significatif sur le microrelief des avant-bras, dès 30 minutes à la dose 0,10% et est maximal à 0,50%. Cet effet se prolonge deux heures après l'application.

Après seulement 1 heure d'application, l'agent selon l'invention formulé à 0,10% tend à lisser le microrelief cutané sur le ventre en diminuant les paramètres de rugosité 3D. Après 14 jours d'applications biquotidiennes,-l'agent selon l'invention lisse le microrelief en diminuant significativement : le paramètre Sa de 9,0% et le paramètre Sq de 9,8%. Cet effet a été observé respectivement chez 89% et 83 % des volontaires l'ayant testé. L'agent selon l'invention présente donc un effet lissant sur le microrelief cutané, cet effet est dose dépendant.

b) Effet de l'agent selon l'invention sur les rides

L'objectif de cette étude a été d'évaluer, *in vivo*, contre placebo l'effet anti-rides de l'agent selon l'invention formulé dans trois types de formules cosmétiques :

- en gel-émulsionné (composition de l'exemple 3) à différentes doses (0,10% et 0,50%), 30 minutes et 2 heures après une application unique au niveau de la patte d'oie;
- en sérum (composition de l'exemple 5) à 1,00%, 30 minutes après application unique au niveau de la patte d'oie ;
- en masque-crème (composition de l'exemple 7) à 0.5%, 30 minutes après une application unique sur les rides du visage et après réalisation de 2 masques-crèmes au cours d'une période de 7 jours.

Dans le cas du gel-émulsionné, l'effet anti-rides a été mesuré grâce à une analyse par projection de franges (système Eotech) au niveau de la patte d'oie et évalué cliniquement sur photographies en aveugle par des experts sur une échelle de score allant de 1 à 6 et basée sur l'évaluation de la profondeur de la ride la plus profonde.

Dans le cas du sérum, l'effet anti-rides a été évalué cliniquement par des experts sur photographies et scoré par les volontaires elles-mêmes.

Dans le cas du masque-crème, l'effet anti-rides a été mesuré grâce à une analyse par projection de franges au niveau des rides du dessus des lèvres.

Effet anti-rides immédiat de l'agent selon l'invention formulé en gel-émulsionné *par projection de franges :*

Les résultats correspondants à l'effet anti-rides de l'agent selon l'invention formulé à 0,10% et 0,50% en gel-émulsionné (composition de l'exemple 3), 30 minutes et 2 heures après une application unique, sont présentés dans le Tableau 11.

| | Variation / Placebo (%) | | | |
|---|---|---|---|---|
| | 30 minutes | | 2 heures | |
| | Agent à 0,10% | Agent à 0,50% | Agent à 0,10% | Agent à 0,50% |
| Paramètre Sa | -4,9% | -5,2% | -5,2% | -6,2% |
| Paramètre Sq | -5,0% | -5,0% | -6,6% | -6,9% |
| Volume négatif | -11,5% | -12,3% | -18,3% | -21,2% |

*Tableau 11*

Dans les conditions de cette étude, l'agent selon l'invention formulé en gel-émulsionné, présente un effet anti-rides immédiat au niveau de la patte d'oie significatif dès 30 minutes. Cet effet se prolonge deux heures après application et est dose-dépendant. Il diminue significativement les paramètres caractéristiques du relief cutané de la patte d'oie. Cet effet a été observé pour la dose :

- 0,10% : chez 68% des volontaires 30 minutes après application, chez 67% des volontaires 2 heures après application ;
- 0,50% : chez 70% des volontaires 30 minutes après application et chez 74% des volontaires 2 heures après application.

Effet anti-rides immédiat de l'agent selon l'invention formulé en gel-émulsionné sur photographies :

Les résultats correspondants à l'effet de l'agent selon l'invention, formulé en gel-émulsionné (composition telle que présentée dans la partie exemple) à différentes doses, sur le stade de rides des pattes d'oie, 30 minutes et 2 heures après une application unique sont présentés dans le Tableau 12.

| | Variation / Placebo (%) | |
|---|---|---|
| | 30 minutes | 2 heures |
| Agent à 0,10% | -6,8% | -8,7% |
| Agent à 0,50% | -7,9% | -12,1% |

*Tableau 12*

Dans les conditions de cette étude, l'agent selon l'invention formulé en gel-émulsionné, permet de réduire significativement le stade de rides des pattes d'oie dès 30 minutes. Cet effet se prolonge deux heures après application et est dose dépendant.

Deux heures après application l'agent selon l'invention à 0,10%, le jury d'experts a noté une amélioration du stade de rides de la patte d'oie dans 55% des cas. Ce pourcentage atteint 63% lorsque l'agent est utilisé à 0,50%.

Effet anti-rides immédiat évalué sur photographies de l'agent selon l'invention formulé en sérum:

Les résultats correspondants à l'effet de l'agent selon l'invention, formulé en sérum (composition telle que présentée dans la partie exemple) à 1%, sur le stade de rides des pattes d'oie, 30 minutes après une application unique sont présentés dans le Tableau 13.

| | Variation / J0 (%) | Variation / Placebo (%) |
|---|---|---|
| Placebo | +1,0% | |
| | | |
| Agent à 1,00% | -9,7% | -10,7% |

*Tableau 13*

Dans les conditions de cette étude, 30 minutes après une application unique, l'agent selon l'invention formulé à 1,00% dans un sérum, réduit de façon significative de 10,7% le stade de rides moyen au niveau des pattes d'oie chez 60% des volontaires.

Par son action rapide, il permet ainsi d'apporter à la peau un effet lifting express.

Dans les conditions de cette étude et seulement 30 minutes après une application unique sur l'ensemble du visage, l'agent selon l'invention formulé à 1,00% dans un sérum permet de diminuer le stade de rides moyen des volontaires.

Cet effet est perçu par les volontaires elles-mêmes qui sont plus nombreuses dans le groupe ayant testé l'agent selon l'invention à trouver que l'agent apporte un effet tenseur immédiat, rend la peau plus ferme et plus lumineuse.

Effet anti-rides par projection de franges de l'agent selon l'invention formulé en masque-crème :

Les résultats correspondants à l'effet de l'agent selon l'invention, formulé à 0,50% en masque-crème (composition telle que présentées dans la partie exemples), sur les rides du dessus des lèvres sont présentés dans le Tableau 14.

| | Variation / Placebo (%) | |
|---|---|---|
| | Après 1 application | Après 3 applications |
| Paramètre Sa | -2,1% | -4,6% |
| Paramètre Sq | -2,9% | -4,6% |
| Volume négatif | -10,7% | -11,9% |

*Tableau 14*

Dans les conditions de cette étude, l'agent selon l'invention formulé à 0,50% en masque-crème diminue les paramètres caractéristiques des rides du dessus des lèvres. Cet effet est visible dès la première application chez 80% des volontaires (-10,7%). Après trois applications, l'efficacité de l'agent s'accentue (-11,9%) et est observée chez 65% des volontaires.

La formule contenant l'agent est perçue globalement comme étant plus efficace que la formule placebo par les volontaires l'ayant testée.

Dès la première application, plus de 90% des sujets ressentent un effet tenseur immédiat, trouvent leur peau plus lisse et plus lumineuse.

Après 3 applications, l'ensemble des sujets trouve leur peau plus hydratée. Elles observent une peau plus lisse et plus douce, et des rides et ridules moins visibles.

c) Effet de l'agent selon l'invention sur les propriétés de surface de la peau L'objectif de cette étude a été d'évaluer, *in vivo,* contre placebo l'effet tenseur de l'agent

selon l'invention, formulé à différentes doses (0,05%, 0,10%, 0,25% et 0,50%) dans un gel-émulsionné (composition de l'exemple 3), 30 minutes et 2 heures après une application isolée.

L'effet tenseur a été mesuré avec le cutomètre Dual MPA 580 (Courage & Khazaka).

Les résultats correspondants à cet effet tenseur sont présentés dans le Tableau 15.

| | Variation / Placebo (%) | | | |
|---|---|---|---|---|
| | 30 minutes | | 2 heures | |
| | Paramètre -Uf | Paramètre –Ue | Paramètre –Uf | Paramètre -U |
| Agent à 0,05% | +3,2% | +4,4% | +5,1% | +5,7% |
| Agent à 0,10% | +3,7% | +6,0% | +5,5% | +6,1% |
| Agent à 0,25% | +4,1% | +6,0% | +6,3% | +7,0% |
| Agent à 0,50% | +6,2% | +9,2% | +7,2% | +10,0% |

*Tableau 15*

Dans les conditions de cette étude et en comparaison au placebo, l'agent selon l'invention présente un effet tenseur significatif dès 30 minutes à la dose 0,05% et est dose-dépendant. Cet effet se prolonge deux heures après l'application.

3) **Effet film perceptible**

Les sensations perçues par les utilisateurs lors de l'application de l'agent selon l'invention ont été analysées.
Des experts sensoriels ont conclu à un effet tenseur important et dose-dépendant du produit. Cet effet est perceptible dans différentes formules *(gel ou fond de teint).* Parallèlement, des volontaires non-experts ont aussi perçu les effets immédiats tenseur et 30 minutes après l'application de l'agent sur le visage.
Les effets visuels de l'agent selon l'invention ont été également mesurés. Il a ainsi été montré que son application permet une amélioration de l'apparence globale du visage qui se traduit par une réduction de la surface des pores et une augmentation de l'éclat du teint. Les autoévaluations face à un miroir ont montré que les volontaires jugent leur peau plus lisse, lumineuse, tendue et hydratée. Enfin, incorporé dans une formule fond de teint, l'agent accroit la tenue du maquillage.
L'effet liftant est perçu par des experts sensoriels et des non-experts. L'invention permet une amélioration globale de l'aspect du visage des volontaires en réduisant la taille des pores et en donnant à la peau un coup d'éclat.

a) Effet tenseur sensoriel de l'agent selon l'invention

L'objectif de cette étude a été de quantifier, *in vivo,* contre placebo :

- sur un panel d'experts sensoriels, les effets tenseur et lissant perçus après application de l'agent selon l'invention formulé à 0,50% dans un fond de teint (composition de l'exemple 6) ;
- sur un panel d'experts sensoriels, l'effet tenseur perçu après application de l'agent selon l'invention formulé à différentes doses en gel (composition de l'exemple 4) ;
- sur un panel non-experts, les effets tenseurs et lissant perçus après application l'agent selon l'invention formulé à 0.10% ou 0.50% en gel-émulsionné (composition de l'exemple 3).

La perception de ces effets a été appréciée sur une échelle de score allant de 1 à 10 (1 :pas de sensation, 10 : sensation tenseur maximale).
Le fond de teint est appliqué par massage léger sur l'ensemble du visage. La notation est réalisée immédiatement, 5mn et 15mn après application, en se basant sur les sensations ressenties et les auto-observations faites dans un miroir sous lumière contrôlée.
Le gel est appliqué par massage léger au niveau de la patte d'oie. La notation est réalisée 3mn, 5mn et 10mn après application du produit.
Le gel-émulsionné est appliqué par massage léger sur le visage entier. Les effets tenseur et lissant ont été mesurés immédiatement, 5, 15 et 30 minutes après application du produit.
Les résultats obtenus sont présentés dans les tableaux 16 à 18.

*Tableau 16*

| Fond de teint | Moyenne des scores (U.A.) | |
|---|---|---|
| | Effet tenseur | Effet lissant |
| Placebo | 0,8 | 0,7 |
| Agent à 0,50% | 1,7 | 1,4 |

Dans les conditions de cette étude, après une application unique, l'agent à 0.5% dans un fond de teint présente des effets tenseur et lissant significatifs supérieurs à celui du placebo.

*Tableau 17*

| Gel | Score moyen (UA) |
|---|---|
| Placebo | 1,8 |
| Agent à 0,05% | 2,4 |
| Agent à 0,10% | 3,5 |
| Agent à 0,25% | 4,2 |
| Agent à 0,50% | 5,1 |

Après application de l'agent selon l'invention formulé en gel à différentes doses sur la zone de la patte d'oie :

- dès la dose 0,10%, 83% des experts sensoriels entraînés à percevoir et à quantifier la sensation de l'effet tenseur ont ressenti un effet tenseur supérieur à l'effet moyen ressenti avec la formule placebo,
- à la dose de 0,50%, ce chiffre atteint 98%.

*Tableau 18*

| Gel-émulsionné | Score moyen (U.A.) | |
|---|---|---|
| | Effet tenseur | Effet lissant |
| Placebo | 2,5 | 3,6 |
| Agent à 0,10% | 3,6 | 4,6 |
| Agent à 0,50% | 3,8 | 4,9 |

Dans les conditions de cette étude, après une application unique, l'agent selon l'invention formulé à 0,10% ou 0,50% en gel-émulsionné, est significativement mieux perçu que la formule placebo par des volontaires non-experts sensoriels. En effet, il est ressenti comme tenseur et lissant par ces volontaires et cet effet est dose-dépendant.

b) Effet de l'agent selon l'invention sur l'apparence globale du visage

L'objectif de cette étude a été d'évaluer, *in vivo,* contre placebo l'effet de l'agent selon l'invention formulé à 0,50% en masque-crème (composition de l'exemple 8), sur l'amélioration globale du visage, 30 minutes après 1 application ou après une cure de 3 masques réalisés sur 7 jours.
L'amélioration globale du visage a été évaluée grâce à :

- une étude de la surface des pores au niveau de la joue par projection de franges (système Eotech) ;
- une évaluation visuelle de l'éclat du teint par des experts ;
- une auto-évaluation de la performance perçue par le volontaire via un questionnaire complété après observation face à un miroir.

Les masques-crèmes (placebo ou agent selon l'invention) ont été appliqués en couche épaisse sur l'ensemble du visage. Les volontaires devaient le laisser agir 20 minutes avant de faire pénétrer l'excédent en massant du bout des doigts.

Les résultats sur la surface des pores correspondants à l'effet de l'agent, formulé à 0,50% en masque-crème, en comparaison à un groupe placebo sont présentés dans le Tableau 19.

**Tableau 19**

|  | Variation / Placebo (%) |
|---|---|
| Après 1 application | -9,2% |
| Après 3 applications | -10,4% |

Dans les conditions de cette étude, dès la première utilisation, l'agent selon l'invention formulé à 0,50% dans un masque-crème améliore significativement le grain de peau en réduisant la surface des pores de 9,2% (effet observé chez 65% des sujets).

Cette diminution atteint 10,4% après 3 applications de masque-crème. Cet effet a été observé chez 70% des volontaires ayant testé la formule contenant l'agent, contre seulement 38% pour celles ayant testé le placebo.

Ainsi, l'agent selon l'invention participe à l'amélioration globale du visage en affinant le grain de peau.

Les résultats sur l'éclat du teint évalué par des experts, correspondants à l'effet de l'agent selon l'invention, formulé à 0,50% en masque-crème, en comparaison à un groupe placebo sont présentés dans le Tableau 20.

|  | Variation / Placebo (%) | |
|---|---|---|
|  | Après 1 application | Après 3 applications |
| Rayonnement | +5,9% | +6,5% |
| Fatigue des yeux | -7,0% | -14,1% |
| Grain de peau | +5,9% | +7,1% |

**Tableau 20**

L'agent selon l'invention améliore significativement l'éclat du teint. Dès la première application, le rayonnement de la peau est augmenté, l'état de fatigue des yeux diminué et le grain de peau affiné.

Les résultats de la performance perçue aux questions fermées de l'auto-évaluation visuelle face à un miroir sont présentés dans le Tableau 21.

**Tableau 21**

|  | Après 1 application | | Après 3 applications | |
|---|---|---|---|---|
| Cumul des réponses «plutôt d'accord» et «d'accord» (%) | Groupe placebo | Groupe Agent à 0,50% | Groupe Placebo | Groupe Agent à 0,50% |
| Ce soin procure un effet tenseur immédiat. | 62 | 91 | 57 | 87 |
| Ce soin hydrate bien la peau. | 90 | 100 | 90 | 100 |
| Avec ce soin, la peau est lissée. | 81 | 91 | 76 | 87 |
| Avec ce soin, la peau est plus lumineuse. | 62 | 91 | 76 | 83 |
| Avec ce soin, la peau est visiblement plus tendue. | 57 | 78 | 62 | 83 |
| Ce soin laisse la peau confortable. | 90 | 96 | 8 | 100 |

La formule contenant l'agent selon l'invention est perçue globalement comme étant plus efficace que la formule placebo par les volontaires l'ayant testée.

Dès la première application, 91% des sujets ressentent un effet tenseur immédiat, trouvent leur peau plus lisse et plus lumineuse.

Après 3 applications de la formule masque-crème, l'ensemble des sujets ayant testé l'agent selon l'invention trouve leur peau plus hydratée et confortable. Elles ont également pu observer une peau plus lisse et visiblement plus tendue.

c) Effet de l'agent selon l'invention sur la tenue du maquillage

L'objectif de cette étude est d'évaluer, *in vivo,* contre placebo l'influence de l'agent selon l'invention formulé à 0,50% sur la tenue dans le temps d'un fond teint (composition de l'exemple 6).

La tenue du fond de teint a été quantifiée sur photographies numériques.

L'agent formulé en fond de teint, a été appliqué le matin en conditions réelles d'utilisation, à domicile par les volontaires. Immédiatement après application puis en fin de journée (en moyenne 11 heures après application), les volontaires ont rempli un questionnaire d'auto-évaluation.

Les résultats correspondants à la perte d'intensité de couleur d'un fond de teint sous l'effet de l'agent selon l'invention formulé à 0,50% dans le temps en comparaison à un placebo sont présentés dans le Tableau 22.

*Tableau 22*

|  | Perte d'intensité de couleur (%) | | |
|---|---|---|---|
|  | 2 heures | 4 heures | 6 heures |
| Placebo | 10% | 15% | 24% |
| Agent à 0,50% | 5% | 11% | 18% |

Dans les conditions de cette étude, l'agent selon l'invention formulé à 0,50% dans un fond de teint permet d'allonger significativement la tenue du maquillage comparé à la formule placebo (+33% de temps gagné). Cet effet a été observé chez 63% des volontaires. Dans ces conditions d'utilisation, l'agent formulé à 0,50% dans un fond de teint est globalement mieux perçu par les utilisatrices qui le préfèrent au fond de teint placebo.

d) Effet de l'agent selon l'invention sur la dispersion des pigments

L'objectif de cette étude est de montrer l'effet de l'agent selon l'invention sur la dispersion des pigments. Pour cela, deux types de pigments habituellement présents dans les produits de maquillage ont été testés : lipophile et hydrophile.

Dans des tubes à essais, 2g de pigment lipophile (OTS-2 Yellow LL) ou hydrophile (Red iron oxide) ont été ajoutée à de l'eau distillée ou aux solutions de 0.5% ou 1% de l'agent selon l'invention. Des photographies ont été prises immédiatement après l'agitation et après au moins 1 heure de repos. Les résultats sont qualitatifs, et ont été évaluées selon 4 niveaux de dispersion des pigments :

- Pas de dispersion-
- Faible dispersion +
- Bonne dispersion ++
- Très bonne dispersion +++

Les résultats sont les suivants :

*Tableau 23*

| Pigment lipophile | Dispersion à T0 | Dispersion à T1h |
|---|---|---|
| Témoin eau | +++ | - |
| Agent selon l'invention 0.5% | +++ | ++ |
| Agent selon l'invention 1% | +++ | ++ |

*Tableau 24*

| Pigment lipophile | Dispersion à T0 | Dispersion à T1h |
|---|---|---|
| Témoin eau | +++ | - |
| Agent selon l'invention 0.5% | +++ | ++ |
| Agent selon l'invention 1% | +++ | ++ |

Dans les conditions de l'étude, l'agent selon l'invention à 0.5% et 1% présente des propriétés de dispersion des pigments.

Ainsi, il participe à la stabilisation des formules et favorise une répartition homogène des pigments à la surface de la peau.

**4) Effet film seconde peau « booster de beauté » :**

Des tests visant à démontrer que des visages traités avec l'agent selon l'invention étaient perçus par des tierces personnes comme plus attractifs ou moins ridés, ont également été mis en place.

Ainsi, les évaluateurs naïfs ont jugé que :

- dans 53% des cas l'agent selon l'invention augmente l'attractivité du visage de volontaires jeunes,

dans 55% des cas, l'agent selon l'invention permet une réduction des rides du visage chez des personnes matures.

Nous avons aussi réalisé deux tests consommateurs en France et en Asie. Les volontaires caucasiens ont appliqué biquotidiennement l'agent selon l'invention en gel émulsionné pendant 14 jours. Les volontaires asiatiques ont réalisé une cure basée sur 6 applications en masque tissu pendant 15 jours.

Ces deux études révèlent que le traitement par l'agent selon l'invention est systématiquement mieux noté que le placebo. Les volontaires caucasiennes déclarent que leur peau est tonifiée, plus ferme comme liftée avec des contours du visage plus nets et un ovale redessiné. Selon les déclarations des volontaires asiatiques, leur teint est plus éclatant et lumineux, leur peau est tonifiée et plus souple.

L'efficacité de l'agent selon l'invention a été confirmée par les utilisatrices elles-mêmes ainsi que par des tierces personnes. Cet agent filmogène agit comme un véritable activateur de beauté en améliorant l'attractivité des visages des personnes jeunes et l'aspect ridée des peaux matures.

En résumé, l'agent selon l'invention a été testé sur deux types de peaux (caucasiennes et asiatiques), après 14 temps d'analyse (instantané, moyen-terme, long-terme) pour 5 doses (0.05% à 1%) et dans 7 formules cosmétiques différentes (émulsion, gel-émulsionné, gel, sérum, fond de teint, masque crème et masque tissu).

a) Effet de l'agent selon l'invention sur l'attractivité perçue par un tiers

L'objectif de cette étude a été d'évaluer, *in vivo,* l'effet de l'agent selon l'invention formulé à 0,50% en masque-crème (composition de l'exemple 8), sur l'amélioration globale du visage perçue par des évaluateurs naïfs.

L'attractivité du visage a été scorée sur photographies numériques.

Les masques-crèmes (placebo ou agent selon l'invention) ont été appliqués en couche épaisse sur l'ensemble du visage. Les volontaires devaient le laisser agir 20 minutes avant de faire pénétrer l'excédent en massant du bout des doigts.

Les panels sont un panel de peaux jeunes (44 volontaires sains d'âge moyen 40 ans) et un panel de peaux matures (40 volontaires sains, d'âge moyen 62 ans).

Les résultats correspondants à l'effet de l'agent selon l'invention formulé à 0,50% en masque-crème sur l'attractivité du visage sur le panel jeune sont présentés dans le Tableau 25, sur les rides du panel mature dans le Tableau 26.

| | Evaluateurs (%) | | | |
|---|---|---|---|---|
| | Placebo | | Agent à 0,50% 10 | |
| | Après 1 application | Après 3 applications | Après 1 application | Après 3 applications |
| Ayant constaté une amélioration | 14 | 25 | 24 | 53 |
| N'ayant pas constaté d'amélioration | 86 | 75 | 76 | 47 |

*Tableau 25*

Dans les conditions de cette étude, après trois applications d'un masque-crème contenant 0,50% de l'agent selon l'invention, des évaluateurs naïfs ont observé une amélioration de l'apparence du visage dans 53% des cas contre seulement 25% des cas pour les sujets ayant testé la formule placebo.

| | Evaluateurs (%) | | | |
|---|---|---|---|---|
| | Placebo | | Agent à 0,50% 25 | |
| | Après 1 application | Après 3 applications | Après 1 application | Après 3 applications |
| Ayant constaté une amélioration | 15 | 25 | 20 | 45 |
| N'ayant pas constaté d'amélioration | 85 | 75 | 80 | 55 |

*Tableau 26*

Dans les conditions de cette étude, après trois applications d'un masque-crème contenant 0,50% de l'agent selon l'invention, des évaluateurs naïfs ont observé une diminution des rides du visage dans 45% des cas contre seulement 25% des cas pour les sujets ayant testé la formule placebo.
L'agent selon l'invention formulé à 0,50% en masque-crème permet donc une diminution visible des rides du visage.

b) Etudes consommateurs de l'agent selon l'invention

Deux études consommateurs ont été menées pour démontrer l'effet de l'agent selon l'invention, une étude sur consommatrices françaises, une étude sur consommatrices asiatiques.
L'objectif du test consommatrices françaises est de comparer l'efficacité d'un soin contenant l'agent selon l'invention formulé à 0,50% en gel-émulsionné (composition de l'exemple 3) à son placebo.
136 femmes vivant en France, d'âge compris entre 40 et 55 ans, ayant tous types de peaux du visage sans quota de sensibilité, déclarant avoir des rides et/ou ridules ainsi qu'un teint terne, manquant d'éclat et utilisatrices de soin tenseur. 68 femmes ont utilisé le placebo et 68 femmes l'agent selon l'invention.
Les sensations observées lors du traitement ont été évaluées au moyen de questionnaires d'auto-évaluation complétés à domicile. Les évaluations ont été réalisées 30 minutes après une première application ainsi qu'au 8ème et 15ème jour de test. Les panélistes ont appliqué le soin sur l'ensemble du visage, sur une peau parfaitement nettoyée, deux fois par jour, matin et soir, pendant 14 jours, à la place de leur soin visage habituel.
Les résultats des questions fermées après la première application sont présentés dans le Tableau 27.

**Tableau 27**

|  | Après 1 application | |
|---|---|---|
|  | Placebo | Agent à 0,50% |
| Ce soin tonifie la peau | 41 | 59 |
| Ce soin rend la peau plus ferme | 35 | 49 |
| Avec ce soin, la peau est visiblement plus tendue | 41 | 54 |
| Ce soin procure un effet tenseur immédiat | 43 | 54 |
| Avec ce soin, la peau est plus douce | 77 | 87 |
| Avec ce soin, la peau est lumineuse | 44 | 49 |
| Avec ce soin, mes pores sont resserrés | 34 | 38 |
| Ce soin minimise les imperfections | 27 | 31 |

Les résultats des questions fermées après 7 jours d'applications biquotidiennes présentés dans le Tableau 28.

**Tableau 28**

|  | Après 7 jours d'application | |
|---|---|---|
|  | Placebo | Agent à 0,50% |
| Ce soin tonifie la peau | 63 | 73 |
| Ce soin rend la peau plus ferme | 69 | 70 |
| Avec ce soin, la peau est visiblement plus tendue | 61 | 69 |
| Ce soin procure un effet tenseur | 69 | 75 |
| Avec ce soin, la peau est plus douce | 79 | 88 |
| Avec ce soin, la peau est lumineuse | 58 | 64 |
| Ce soin minimise les imperfections | 48 | 60 |
| Avec ce soin, les contours du visage paraissent plus nets | 36 | 49 |
| Avec ce soin, l'ovale du visage se redessine | 24 | 39 |

Les résultats des questions fermées après 14 jours d'applications biquotidiennes sont présentés dans le Tableau 29.

**Tableau 29**

|  | Après 14 jours d'application | |
|---|---|---|
|  | Placebo | Agent à 0,50% |
| Ce soin tonifie la peau | 67 | 76 |
| Ce soin rend la peau plus ferme | 76 | 84 |
| Avec ce soin, la peau est visiblement plus tendue | 63 | 73 |
| Ce soin procure un effet tenseur | 72 | 78 |
| Avec ce soin, la peau est comme liftée | 54 | 63 |
| Avec ce soin, la peau est plus douce | 85 | 93 |
| Avec ce soin, la peau est lumineuse | 64 | 72 |
| Ce soin minimise les imperfections | 55 | 58 |
| Avec ce soin, les contours du visage paraissent plus nets | 48 | 63 |

(suite)

| | Après 14 jours d'application | |
|---|---|---|
| | Placebo | Agent à 0,50% |
| Avec ce soin, l'ovale du visage se redessine | 34 | 58 |

Les résultats des commentaires libres après 14 jours d'applications biquotidiennes sont présentés dans le tableau 30.

*Tableau 30*

| | Après 14 jours d'application | |
|---|---|---|
| Commentaires libres | Placebo | Agent à 0,50% |
| Effet lissant immédiat, peau lisse, moins froissée | 9 | 16 |
| Peau plus lumineuse, moins terne, éclatante | 16 | 22 |
| Pores resserrés, grain de peau affiné | 7 | 12 |
| Effet tenseur immédiat | 16 | 19 |
| Peau plus ferme (*) | 2 | 10 |
| Peau plus belle, améliorée | 9 | 16 |

D'une manière globale, dans les conditions de cette étude, les femmes ayant utilisé la formule contenant l'agent selon l'invention ont attribué de meilleurs résultats pour chaque item que celles ayant testé la formule placebo.

Il y a significativement plus de volontaires ayant trouvé leur peau plus tonifiée, plus ferme, comme liftée avec des contours du visage plus nets et un ovale redessiné.

L'objectif du test consommatrices asiatiques est de comparer l'efficacité d'un masque tissu (composition présentée dans la partie exemples) contenant l'agent selon l'invention formulé à 0,50% ou une formule placebo.

134 femmes asiatiques, d'âge compris entre 30 et 50 ans vivant à Singapour, ayant tous types de peaux du visage sans quota de sensibilité, déclarant avoir des rides et/ou ridules ainsi qu'un teint terne, manquant d'éclat et utilisatrices de masques destinés au soin du visage. 66 femmes ont testé l'agent selon l'invention et 68 femmes le placebo.

Les sensations observées lors du traitement ont été évaluées au moyen de questionnaires d'auto-évaluation complétés à domicile. Les évaluations ont été réalisées après application du premier masque et après application des 5 autres masques. Les masques ont été effectués à J0, J3, J6, J9, J12 et J15.

Les résultats des questions fermées après 1 application sont présentés dans le Tableau 31.

*Tableau 31*

| | Après 1 application | |
|---|---|---|
| | Placebo | Agent à 0,50% |
| Ce soin hydrate bien la peau | 46 | 61 |
| Ce soin laisse la peau souple | 44 | 68 |
| Ce soin tonifie la peau | 40 | 47 |
| Avec ce soin, la peau est lumineuse | 34 | 38 |
| Avec ce soin, la peau est vitalisée | 38 | 45 |
| Avec ce soin, le teint est éclatant | 40 | 44 |
| Avec ce soin, mes pores sont resserrés | 32 | 41 |
| Avec ce soin, la peau est lissée | 49 | 59 |
| Ce soin procure un effet tenseur immédiat | 56 | 62 |
| Avec ce soin, la peau est visiblement plus tendue | 38 | 42 |

Les résultats des questions fermées après 6 applications sont présentés dans le Tableau 32.

**Tableau 32**

| | Après 6 applications | |
|---|---|---|
| | Placebo | Agent à 0,50% |
| Ce soin hydrate bien la peau | 70 | 79 |
| Ce soin laisse la peau souple | 71 | 86 |
| Ce soin tonifie la peau | 64 | 80 |
| Avec ce soin, la peau est lumineuse | 64 | 74 |
| Avec ce soin, la peau est vitalisée | 68 | 80 |
| Avec ce soin, le teint est éclatant | 62 | 77 |
| Avec ce soin, mes pores sont resserrés | 65 | 73 |
| Avec ce soin, les rides et ridules sont atténuées | 56 | 61 |
| Avec ce soin, la peau est visiblement plus tendue | 65 | 70 |

D'une manière globale, dans les conditions de cette étude, les femmes ayant utilisé les masques imbibés avec la lotion contenant l'agent selon l'invention ont donné de meilleurs résultats pour chaque item que celles ayant testé la formule placebo.

Il y a significativement plus de volontaires ayant trouvé leur teint plus éclatant et plus lumineux ainsi que leur peau plus tonifiée et plus souple.

**Revendications**

1. Agent cosmétique ou dermocosmétique, constitué de galactomannanes obtenus à partir de *Caesalpinia spinosa* de masses molaires comprises entre 1 et 150kDa et présentant une masse molaire moyenne comprise entre 5 et 30kDa, de galactanes sulfatés réticulés obtenus à partir de *Kappaphycus alvarezii* de masses molaires comprises entre 1 et 150kDa et présentant une masse molaire moyenne comprise entre 5 et 25kDa, et d'eau.

2. Agent cosmétique ou dermocosmétique, constitué de galactomannanes obtenus à partir de *Caesalpinia spinosa* de masses molaires comprises entre 1 et 150kDa, et de galactanes sulfatés réticulés obtenus à partir de *Kappaphycus alvarezii* de masses molaires comprises entre 7 et 40kDa.

3. Agent cosmétique ou dermocosmétique selon l'une des précédentes revendications, **caractérisé en ce que** les galactomannanes obtenus à partir de *Caesalpinia spinosa* présentent une masse molaire moyenne comprise entre 8 et 25kDa, et les galactanes sulfatés réticulés obtenus à partir de *Kappaphycus alvarezii* présentent une masse molaire moyenne comprise entre 8 et 20kDa.

4. Agent cosmétique ou dermocosmétique selon l'une des précédentes revendications, **caractérisé en ce que** les galactomannanes sont obtenus par hydrolyse de galactomannanes de *Caesalpinia spinosa.*

5. Agent cosmétique ou dermocosmétique selon l'une des précédentes revendications, **caractérisé en ce que** les galactanes sulfatés sont obtenus par hydrolyse de galactanes sulfatés de *Kappaphycus alvarezii.*

6. Agent cosmétique ou dermocosmétique selon l'une des précédentes revendications, **caractérisé en ce que** les galactanes sulfatés sont réticulés avec un agent de réticulation de nature ionique.

7. Agent cosmétique ou dermocosmétique selon l'une des précédentes revendications, **caractérisé en ce qu'**il est constitué de :

- 60 à 90% de galactomannanes, et
- 10 à 40% de galactanes sulfatés réticulés,

les pourcentages étant donnés en masse/masse.

8. Agent cosmétique ou dermocosmétique selon l'une des précédentes revendications, **caractérisé en ce qu'**il est constitué de :

- 70 à 90% de galactomannanes, et
- 10 à 30% de galactanes sulfatés réticulés,

les pourcentages étant donnés en masse/masse.

9. Agent cosmétique ou dermocosmétique selon l'une des précédentes revendications, **caractérisé en ce que** les galactomannanes et les galactanes sulfatés réticulés forment ensemble un réseau interpénétré.

10. Utilisation cosmétique non thérapeutique d'un agent selon l'une des précédentes revendications, comme agent cosmétique tenseur et/ou filmogène.

11. Utilisation cosmétique non thérapeutique d'un agent selon l'une des revendications 1-9, pour la protection de la peau.

12. Agent cosmétique ou dermocosmétique selon l'une des revendications 1-9, pour son utilisation dans la protection de la peau contre la pénétration de molécules toxiques.

13. Agent cosmétique ou dermocosmétique selon l'une des revendications 1-9, pour une utilisation dans la protection de la peau contre l'adhésion bactérienne de pathogènes.

14. Utilisation cosmétique non thérapeutique d'un agent selon l'une des revendications 1 à 9, comme agent cosmétique pour améliorer l'effet barrière de la peau.

15. Utilisation cosmétique non thérapeutique d'un agent selon l'une des revendications 1 à 9, pour améliorer l'éclat de la peau et/ou lisser le microrelief de la peau et/ou lisser les rides.

16. Utilisation cosmétique non thérapeutique d'un agent selon l'une des revendications 1 à 9, pour lutter contre les manifestations disgracieuses du vieillissement de la peau.

17. Utilisation cosmétique non thérapeutique d'un agent selon l'une des revendications 1 à 9, pour favoriser la tenue du maquillage et/ou améliorer la dispersion des pigments dans les formulations cosmétiques.

18. Utilisation cosmétique non thérapeutique d'un agent selon l'une des revendications 1 à 9, pour un effet seconde peau, booster de beauté.

19. Composition cosmétique ou dermocosmétique adaptée à une application en topique sur la peau humaine comprenant au moins 0.1% d'un agent selon l'une des revendications 1 à 9.

20. Procédé cosmétique ou dermocosmétique pour améliorer l'éclat de la peau et/ou lutter contre les manifestations disgracieuses du vieillissement de la peau, **caractérisé en ce qu'**il consiste à appliquer sur la peau une composition cosmétique selon la revendication 19.

**Patentansprüche**

1. Kosmetisches oder dermokosmetisches Mittel, bestehend aus aus *Caesalpinia spinosa* gewonnenen Galactomannanen mit molaren Massen zwischen 1 und 150 kDa und einer durchschnittliche molaren Masse zwischen 5 und 30 kDa, aus aus *Kappaphycus alvarezii* gewonnenen, vernetzten sulfatierten Galactanen mit molaren Massen zwischen 1 und 150 kDa und einer durchschnittlichen molaren Masse zwischen 5 und 25 kDa und Wasser.

2. Kosmetisches oder dermokosmetisches Mittel, bestehend aus aus *Caesalpinia spinosa* gewonnenen Galactomannanen mit molaren Massen zwischen 1 und 150 kDa und aus *Kappaphycus alvarezii* gewonnenen, vernetzten sulfatierten Galactanen mit molaren Massen zwischen 7 und 40 kDa.

**3.** Kosmetisches oder dermokosmetisches Mittel nach einem der vorhergehenden Ansprüche, **dadurch gekennzeichnet, dass** die aus *Caesalpinia spinosa* gewonnenen Galactomannane eine durchschnittliche molare Masse zwischen 8 und 25 kDa aufweisen und die aus *Kappaphycus alvarezii* gewonnenen, vernetzten sulfatierten Galactane eine durchschnittliche molare Masse zwischen 8 und 20 kDa aufweisen.

**4.** Kosmetisches oder dermokosmetisches Mittel nach einem der vorhergehenden Ansprüche, **dadurch gekennzeichnet, dass** die Galactomannane durch Hydrolyse von Galactomannanen aus *Caesalpinia spinosa* gewonnen werden.

**5.** Kosmetisches oder dermokosmetisches Mittel nach einem der vorhergehenden Ansprüche, **dadurch gekennzeichnet, dass** die sulfatierten Galactane durch Hydrolyse von sulfatierten Galactanen aus *Kappaphycus alvarezii* gewonnen werden.

**6.** Kosmetisches oder dermokosmetisches Mittel nach einem der vorhergehenden Ansprüche, **dadurch gekennzeichnet, dass** die sulfatierten Galactane mit einem Vernetzungsmittel ionischer Natur vernetzt sind.

**7.** Kosmetisches oder dermokosmetisches Mittel nach einem der vorhergehenden Ansprüche, **dadurch gekennzeichnet, dass** es aus Folgendem besteht:

   - 60 bis 90 % Galactomannanen, und
   - 10 bis 40 % vernetzten sulfatierten Galactanen,
   wobei die Prozentsätze in Masse/Masse angegeben sind.

**8.** Kosmetisches oder dermokosmetisches Mittel nach einem der vorhergehenden Ansprüche, **dadurch gekennzeichnet, dass** es aus Folgendem besteht:

   - 70 bis 90 % Galactomannanen, und
   - 10 bis 30 % vernetzten sulfatierten Galactanen,
   wobei die Prozentsätze in Masse/Masse angegeben sind.

**9.** Kosmetisches oder dermokosmetisches Mittel nach einem der vorhergehenden Ansprüche, **dadurch gekennzeichnet, dass** die Galactomannane und vernetzten sulfatierten Galactane zusammen ein miteinander verflochtenes Netzwerk bilden.

**10.** Nichttherapeutische kosmetische Verwendung eines Mittels nach einem den vorhergehenden Ansprüchen als straffendes und/oder filmbildendes kosmetisches Mittel.

**11.** Nichttherapeutische kosmetische Verwendung eines Mittels nach einem der Ansprüche 1 bis 9 zum Schutz der Haut.

**12.** Kosmetisches oder dermokosmetisches Mittel nach einem der Ansprüche 1 bis 9 zum Verwenden beim Schutz der Haut vor dem Eindringen toxischer Moleküle.

**13.** Kosmetisches oder dermokosmetisches Mittel nach einem der Ansprüche 1 bis 9 zum Verwenden beim Schutz der Haut vor bakterieller Adhäsion von Krankheitserregern.

**14.** Nichttherapeutische kosmetische Verwendung eines Mittels nach einem der Ansprüche 1 bis 9 als kosmetisches Mittel zum Verbessern der Barrierewirkung der Haut.

**15.** Nichttherapeutische kosmetische Verwendung eines Mittels nach einem der Ansprüche 1 bis 9 zum Verbessern des Teints der Haut und/oder Glätten des Mikroreliefs der Haut und/oder Glätten von Falten.

**16.** Nichttherapeutische kosmetische Verwendung eines Mittels nach einem der Ansprüche 1 bis 9 zum Bekämpfen der unschönen Erscheinungen der Hautalterung.

**17.** Nichttherapeutische kosmetische Verwendung eines Mittels nach einem der Ansprüche 1 bis 9, um das Anhaften von Make-up zu fördern und/oder die Dispersion von Pigmenten in kosmetischen Formulierungen zu verbessern.

**18.** Nichttherapeutische kosmetische Verwendung eines Mittels nach einem der Ansprüche 1 bis 9 als Beauty-Booster mit einem Effekt als zweite Haut.

**19.** Kosmetische oder dermokosmetische Zusammensetzung, die zur topischen Anwendung auf menschlicher Haut geeignet ist, umfassend mindestens 0,1 % eines Mittels nach einem der Ansprüche 1 bis 9.

**20.** Kosmetisches oder dermokosmetisches Verfahren zum Verbessern des Teints der Haut und/oder zur Bekämpfung der unschönen Anzeichen der Hautalterung, **dadurch gekennzeichnet, dass** es darin besteht, eine kosmetische Zusammensetzung nach Anspruch 19 auf die Haut aufzutragen.

**Claims**

**1.** Cosmetic or dermocosmetic agent, consisting of galactomannans obtained from *Caesalpinia spinosa* having molar masses of between 1 and 150 kDa and having an average molar mass of between 5 and 30 kDa, crosslinked sulfated galactans obtained from *Kappaphycus alvarezii* having molar masses of between 1 and 150 kDa and having an average molar mass of between 5 and 25 kDa, and water.

**2.** Cosmetic or dermocosmetic agent, consisting of galactomannans obtained from *Caesalpinia spinosa* having molar masses of between 1 and 150 kDa, and crosslinked sulfated galactans obtained from *Kappaphycus alvarezii* having molar masses of between 7 and 40 kDa.

**3.** Cosmetic or dermocosmetic agent according to either of the preceding claims, **characterized in that** the galactomannans obtained from *Caesalpinia spinosa* have an average molar mass of between 8 and 25 kDa, and the crosslinked sulfated galactans obtained from *Kappaphycus alvarezii* have an average molar mass of between 8 and 20 kDa.

**4.** Cosmetic or dermocosmetic agent according to any of the preceding claims, **characterized in that** the galactomannans are obtained by hydrolyzing galactomannans of *Caesalpinia spinosa.*

**5.** Cosmetic or dermocosmetic agent according to any of the preceding claims, **characterized in that** the sulfated galactans are obtained by hydrolyzing sulfated galactans of *Kappaphycus alvarezii.*

**6.** Cosmetic or dermocosmetic agent according to any of the preceding claims, **characterized in that** the sulfated galactans are crosslinked with an ionic crosslinking agent.

**7.** Cosmetic or dermocosmetic agent according to any of the preceding claims, **characterized in that** it consists of:

- 60 to 90% galactomannans, and
- 10 to 40% crosslinked sulfated galactans,

the percentages being given by mass/mass.

**8.** Cosmetic or dermocosmetic agent according to any of the preceding claims, **characterized in that** it consists of:

- 70 to 90% galactomannans, and
- 10 to 30% crosslinked sulfated galactans,

the percentages being given by mass/mass.

**9.** Cosmetic or dermocosmetic agent according to any of the preceding claims, **characterized in that** the galactomannans and the crosslinked sulfated galactans together form an interpenetrating network.

**10.** Non-therapeutic cosmetic use of an agent according to any of the preceding claims, as a tightening and/or film-forming cosmetic agent.

**11.** Non-therapeutic cosmetic use of an agent according to any of claims 1 to 9, for protecting the skin.

**12.** Cosmetic or dermocosmetic agent according to any of claims 1 to 9, for its use in protecting the skin against the penetration of toxic molecules.

13. Cosmetic or dermocosmetic agent according to any of claims 1 to 9, for use in protecting the skin against bacterial adhesion of pathogens.

14. Non-therapeutic cosmetic use of an agent according to any of claims 1 to 9, as a cosmetic agent for improving the barrier effect of the skin.

15. Non-therapeutic cosmetic use of an agent according to any of claims 1 to 9, for improving the radiance of the skin and/or smoothing the microrelief of the skin and/or smoothing wrinkles.

16. Non-therapeutic cosmetic use of an agent according to any of claims 1 to 9, for combating the unsightly manifestations of aging of the skin.

17. Non-therapeutic cosmetic use of an agent according to any of claims 1 to 9, for promoting the hold of makeup and/or improving the dispersion of pigments in cosmetic formulations.

18. Non-therapeutic cosmetic use of an agent according to any of claims 1 to 9, for a second skin effect, a beauty booster.

19. Cosmetic or dermocosmetic composition suitable for topical application to human skin, comprising at least 0.1% of an agent according to any of claims 1 to 9.

20. Cosmetic or dermocosmetic method for improving the radiance of the skin and/or combating the unsightly manifestations of aging of the skin, **characterized in that** it consists in applying a cosmetic composition according to claim 19 to the skin.

RID1A,Refractive index Signal (U\2DATA\1082\\SUCRE012.O)

DP18  DP6

DP45  DP2

Echelle abscisses : entre 0, 5, 10, 15, 20, 25, 30, 35 mU
Echelle ordonnées : entre 0, 200, 400, 600, 800, 1000, 1200, 1400 nRU

Figure 1A

RID1A, Refractive Index Signal Y:\HPCHEM\2\DATA\150227\SUCRE021.D)

Echelle abscisses : entre 0, 5, 10, 15, 20, 25, 30, 35 mU
Echelle ordonnées : entre -1000, 0, 1000, 2000, 3000, 4000, 5000, 6000, 7000 nRU

Figure 1B

EP 3 407 866 B1

Echelle abscisses : entre 0, 5, 10, 15, 20, 25, 30, 35 mU
Echelle ordonnées : entre 0, 2000, 4000, 6000, 8000, 10000, 12000, 14000, 16000 nRU

Figure 1C

Efficacité ++++

Figure 2A

Efficacité +++

Figure 2B

Efficacité ++

Figure 2C

Efficacité +

Figure 2D

Efficacité -

Figure 2E

Figure 3

Figure 4A

Figure 4B

EP 3 407 866 B1

Figure 4C

Figure 5A

Figure 5B

Figure 5C

EP 3 407 866 B1

Figure 5D

Figure 6A

Figure 6B

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2881349
- FR 2986430
- EP 0730867 A
- WO 9819663 A
- FR 3018448
- WO 8404039 A
- FR 2882366
- EP 1944065 A

**Littérature non-brevet citée dans la description**

- **MD GUIRY et al.** Kappaphycus alvarezii (Doty) Doty ex P.C. Silva : : Algaebase. *AlgaeBase,* 26 Février 2013, 1-4